(19) Europäisches Patentamt
European Patent Office
Office européen des brevets

(11) EP 3 967 331 A1

(12) EUROPEAN PATENT APPLICATION

(43) Date of publication:
16.03.2022 Bulletin 2022/11

(21) Application number: 20306027.2

(22) Date of filing: 14.09.2020

(51) International Patent Classification (IPC):
*A61K 49/00* (2006.01) *G01N 21/64* (2006.01)

(52) Cooperative Patent Classification (CPC):
A61K 49/0054; A61K 49/0021; A61K 49/0082

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
- Centre national de la recherche scientifique 75016 Paris (FR)
- Université de Strasbourg 67000 Strasbourg (FR)

(72) Inventors:
- COLLOT, Mayeul 67400 ILLKIRCH (FR)
- KLYMCHENKO, Andrey 67400 ILLKIRCH (FR)

(74) Representative: Lavoix
2, place d'Estienne d'Orves
75441 Paris Cedex 09 (FR)

# (54) ULTRA-SMALL FLUORESCENT ORGANIC NANOPARTICLES

(57) The present invention relates to polymeric nanoparticle (NP) having a core and a shell, wherein said NP consists of a polymeric molecule having a lipophilic unit and a unit comprising a polyether (amphiphilic polymer), said lipophilic unit forming said core and said unit comprising a polyether forming said shell. In one embodiment, such polymeric nanoparticles are monomolecular.

In particular, the present invention relates to a polymeric nanoparticle (NP) having a core and a shell, wherein said NP comprises or consists of 1 to 10 polymeric molecules having a lipophilic unit and a unit comprising a polyether, said lipophilic unit forming said core and said unit comprising a polyether forming said shell, wherein said polymeric molecule has the following formula (A):

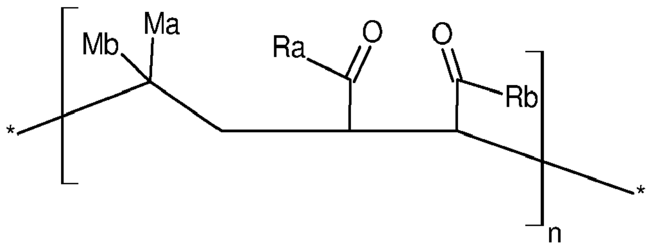

**(A)**

The present invention also relates to the preparation and applications of such polymeric nanoparticles, in particular as pharmaceutical compositions or composition for diagnosis.

EP 3 967 331 A1

## Description

The present invention relates to organic nanoparticles (NPs). The present invention relates to a fluorescent organic nanoparticles (FONs).

In particular, the present invention relates to the preparation of NPs or FONs, their properties and their applications.

## Prior art

Fluorescent organic nanoparticles (FONs) are attractive alternative to the less biocompatible inorganic based nanoparticles. However, several challenges need to be addressed especially the control of their size, their homogeneity, their brightness and their non-specific interactions.

It is notably important to have access to ultra-small nanoparticles, i.e. a size similar to protein size, so biomolecules can be followed with minimal perturbation and allow super-resolution studies. Nanoparticles (NPs) of small size become more and more important for *in vivo* applications due to their better penetration into target tumors, proper elimination and lower propensity to accumulate into liver (- Cabral, H.; Matsumoto, Y.; Mizuno, K.; Chen, Q.; Murakami, M.; Kimura, M.; Terada, Y.; Kano, M. R.; Miyazono, K.; Uesaka, M.; Nishiyama, N.; Kataoka, K. Accumulation of sub-100 nm polymeric micelles in poorly permeable tumours depends on size. Nature Nanotechnology 2011, 6, 815. Zhang, C.; Li, C.; Liu, Y.; Zhang, J.; Bao, C.; Liang, S.; Wang, Q.; Yang, Y.; Fu, H.; Wang, K.; Cui, D. Gold Nanoclusters-Based Nanoprobes for Simultaneous Fluorescence Imaging and Targeted Photodynamic Therapy with Superior Penetration and Retention Behavior in Tumors. Adv. Funct. Mater. 2015, 25, 1314). Quantum dots (QDs) are among the smallest particles, but still are superior to 20 nm with hydrodynamic shell. Smallest dye-loaded SiNPs of about 10 nm core are also around 20 nm hydrodynamic diameter due to large PEG shell. All organic systems exploiting self-assembly suggest effective approaches in fabrication of ultra-small fluorescent NPs. Such systems are for example micellar NPs composed of amphiphilic molecules coupled to a fluorophore. However, it is important that the micelle is polymerized in order to prevent its dissociation in biological media. In these cases, after polymerization, the micelles are composed of polymer chains which ensures a high stability in biological media. An alternative approach that mimics micellar assembly is to directly use an amphiphilic polymer that can fold as a single molecule into micelle-like structure.

PMAO is a low-cost amphiphilic polymer widely used to wrap nanocrystals including QDs, iron and gold nanoparticles in order to make them water-soluble (Di Corato, R.; Quarta, A.; Piacenza, P.; Ragusa, A.; Figuerola, A.; Buonsanti, R.; Cingolani, R.; Manna, L.; Pellegrino, T. Water solubilization of hydrophobic nanocrystals by means of poly(maleic anhydride-alt-1-octadecene). J. Mater. Chem. 2008, 18, 1991). Although PMAO was already used to form dye-including NPs, the dye (squaraine) was encapsulated and non-covalently attached with a risk of dye leakage. Although small NPs (16.2 $\pm$ 6.1 nm) were initially obtained, glycol chitosan coating doubled the diameter to 32.5 $\pm$ 7.5 nm and provided cell-penetrating NPs via non-specific interactions. However, making such NPs fluorescent requires NPs modification with multiple fluorophores, which commonly leads to aggregation-caused quenching (Reisch, A.; Klymchenko, A. S. Fluorescent Polymer Nanoparticles Based on Dyes: Seeking Brighter Tools for Bioimaging. Small 2016, 12, 1968). Therefore, appropriate design of fluorophore should be made to minimize aggregation-caused quenching (ACQ) phenomena, such as introduction of bulky groups, exploiting aggregation-induced emission (AIE) and use of bulky hydrophobic counter-ions in case of ionic dyes (Reisch, A.; Didier, P.; Richert, L.; Oncul, S.; Arntz, Y.; Mely, Y.; Klymchenko, A. S. Collective fluorescence switching of counterion-assembled dyes in polymer nanoparticles. Nat. Commun. 2014, 5, 4089).

## Aims of the invention

The present invention aims to solve the technical problem of providing monomolecular nanoparticles formed by self-folding of an amphiphilic polymer.

The present invention aims to solve the technical problem of providing nanoparticles having of small and uniform size, and preferably a size of less than 35 nm or even less than 20 nm, and preferably less than 15 nm.

The present invention aims to solve the technical problem of providing nanoparticles having fluorescent properties.

The present invention aims to solve the technical problem of providing nanoparticles having a high brightness value (fluorescence quantum yield $\times$ extinction coefficient), typically higher than 1,000,000 $M^{-1}.cm^{-1}$.

The present invention also aims to solve the technical problem of providing such nanoparticles with reduced non-specific interactions in biological media.

The present invention also aims to solve the technical problem of providing nanoparticles targeting a specific target, such as for example a membrane receptor.

The present invention also aims to solve the technical problem of providing stable nanoparticles in biological

media.

**Detailed description of the invention**

The present invention relates to nanoparticles solving one or more of the technical problem explained or suggested by the present invention.

The present invention relates to a monomolecular polymeric nanoparticle (NP) having a core and a shell, wherein said NP consists of a polymeric molecule having a lipophilic unit and a unit comprising a polyether (amphiphilic polymer), said lipophilic unit forming said core and said unit comprising a polyether forming said shell.

The present invention relates in particular to a polymeric nanoparticle (NP) having a core and a shell, wherein said NP comprises or consists of 1 to 10 polymeric molecules having a lipophilic unit and a unit comprising a polyether, said lipophilic unit forming said core and said unit comprising a polyether forming said shell, wherein said polymeric molecule has the following formula (A):

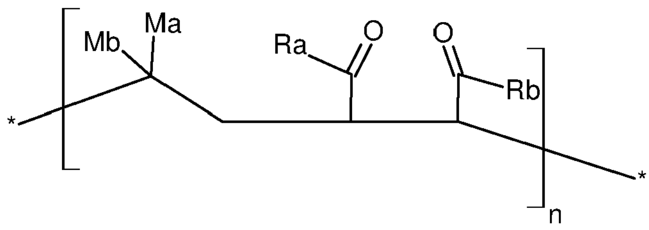

**(A)**

wherein

- n is ranging from 2 to 10,000;
- * represents at each occurrence a radical of a polymerisation initiator;
- Ma and Mb are each independently a hydrogen atom, a, linear or branched, alkyl group having from 1 to 30 carbon atoms, for example from 8 to 30, preferably from 10 to 25, and preferably from 10 to 20, and has for example 16 carbon atoms, or an aryl, for example a benzyl ;Ra and Rb, independently of each other, are OH or a group having the following formula (B):

  -NH - Rc **(B)**

  wherein, the radicals Rc, independently of each other, are:
- a labelling moiety or a targeting moiety; or
- represent a group of formula (C):

  -X-Y **(C)**

  wherein said labelling moiety or said targeting moiety and said group of formula (C) are preferably both present in said polymeric molecule;
  wherein

  - X comprises a polyether;

  and

  - Y is selected from the group consisting of: H, OH, $OCH_3$, $CH_3$, $NH_2$, SH, a ligand, and a functional group ($N_3$, alkyne, strained alkyne like DBCO, dienophile, maleimide, able to bind a ligand; and

  wherein the percentage of the radicals Rc being of formula (C) is ranging from 5% to 100%, preferably from 10% to 100%, based on the total number of the radicals Ra and Rb in said compound of formula (A).

The present invention relates to a core-shell polymeric nanoparticle consisting of 2 to 10 polymeric molecules, typically of structure (A). In an embodiment, said NP has from 2 to 5 polymeric molecules. In an embodiment, said NP

has 2 or 3 polymeric molecules.

The present invention relates to a core-shell polymeric nanoparticle consisting of single polymeric molecule, defined as "monomolecular polymeric nanoparticle". In an embodiment, said NP has 1 or 2 polymeric molecules, and typically from 1 to below 1.5 polymeric molecules (in average). According to the invention the nanoparticle is deemed "monomolecular" when the number of polymer molecules in the NP is below 1.5.

Advantageously, the nanoparticle is formed by self-folding of said polymeric molecule(s).

More specifically, the polymeric molecule has the formula (A) as defined above, said polymeric nanoparticle having a star structure (N) (see figure 13) with a central core and branches in the shell.

In one embodiment, said polymeric molecule has:

- a core, which is lipophilic and comprises in particular the alkyl chains of the compound of formula (A) $(CH_2)_p$); and
- a shell surrounding the core, wherein said shell comprises said radicals Rc.

The present invention also relates to a polymeric nanoparticle consisting of 1 to 10 polymeric molecules having the formula (A) as defined above, said polymeric nanoparticle having the following structure:

- a core, which is lipophilic and comprises in particular the alkyl chains of the compound of formula (A);
- a shell surrounding said core, wherein said shell comprises:
- radicals Rc comprising labelling moieties close to or in contact with the core; and/or
- radicals Rc of formula (C) at the interface of the nanoparticle with the aqueous medium.

In one embodiment, said polymeric molecule comprises or consists of the following structure:

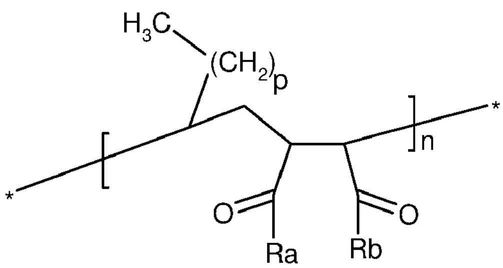

wherein p ranges from 1 to 30, for example from 8 to 30, preferably from 10 to 25, and preferably from 10 to 20, and is for example 15, wherein *, n, Ra and Rb are as defined according to the present invention.

In one embodiment, the percentage of the radicals Rc being of formula (C) is ranging from 40% to 100%, preferably from 50% to 100%, based on the total number of the radicals Ra and Rb in said compound of formula (A) (referred to as "PEG loading 5%)" when X in Rc is a PEG radical or derivative).

In one embodiment, the percentage of the radicals Rc being of formula (C) is ranging from 80% to 100%, preferably from 90% to 100%, based on the total number of the radicals Ra and Rb in said compound of formula (A).

In one embodiment, the percentage of the radicals Rc being of formula (C) is of about 100%.

In one embodiment, the percentage of the radicals Rc being a labelling moiety or a targeting moiety is ranging from 1 to 50%, preferably from 20 to 50%, based on the total number of Ra and Rb radicals in said compound of formula (A).The percentage of the radicals Rc is calculated by the amount of NH2-Rc involved in the reaction compared to the amount of repeating units of the starting polymer (for example PMAO) and the final percentage of the radicals Rc is evaluated by NMR.

In one embodiment, wherein when Rc is a group of formula (C), Rc has the following formula (D):

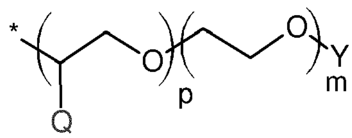
(D)

wherein:

p is ranging from 0 to 10, for example p is ranging from 1 to 8, for example p is 3 or 6;
m is ranging from 15 to 300, for example m is ranging from 10 to 50, for example m is raging from 10 to 35;

Q is H or $CH_3$;
wherein * represents the covalent bond to -NH in Rc
wherein Y is as defined in the present invention.

Such a structure (D) covers compounds wherein the "m" ethylene oxide units are at the terminal end linked to Y and wherein the "p" units are ethylene oxide units, propylene oxide units or a mixture of ethylene oxide and propylene oxide units.

Preferably, when Rc has formula (C), X is chosen among PEG derivatives. More preferably, said PEG derivative is a PEG having a molecular mass Mw of 1,000 $g.mol^{-1}$ or more.

In one embodiment, Rc represents a labelling moiety or a mixture of molecules having formula (D) wherein m is 6 and y is 35, wherein Q is a mixture of H for (EO), or $CH_3$ for (PO) units.

In one embodiment, Rc has one of the following formulae:

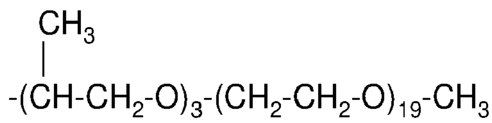

In one embodiment, Rc represents a labelling moiety or the formulae (D) wherein Q is $CH_3$, m is 3 and p is 19.

In one embodiment, the polymeric molecule is a PEGylated PMAO (poly(maleic anhydride-alt-1-octadecene) (PMAO) with polyethylene glycol (PEG)), i.e. said polymeric molecule is a polyethylene glycolated poly(maleic anhydride-alt-1-octadecene).

In a particular embodiment, when Rc has formula (C), the X-Y group corresponds to the Jeffamine® commercialized by Huntsman®, such as JEFFAMINE® M-1000 polyetheramine and JEFFAMINE® M-2070 polyetheramine.

In one embodiment, the radicals Rc, independently of each other, are:

- a fluorophore moiety; or
- have the following group of formula (C):

-X-Y (C),

wherein X and Y are as defined in the present invention,
wherein said fluorophore moiety and said group of formula (C) are preferably both present in said polymeric molecule.

In one embodiment, said fluorophore moiety is selected from the group consisting of:

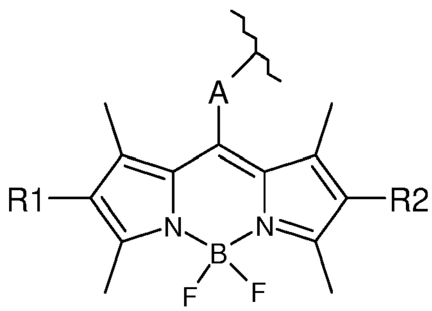

wherein A is a chain comprising carbon atoms and one or more heteroatoms, such as one or more chemical functions -NH-, -O-, -C(=O)-, -C(=O)-NH-, and optionally comprising one or more aromatic groups either in the main chain or as pendant groups,
wherein R1 and R2 are each independently H, an alkyl group, an aromatic group, an alkylaryl group or an aralkyl group, such as for example a methyl, ethyl of phenyl or phenylphenyl group, wherein

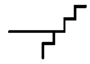

represents a covalent bond to -NH group of formula (B).

In one embodiment, the fluorophore group having or containing one of the following structures or a derivative thereof:

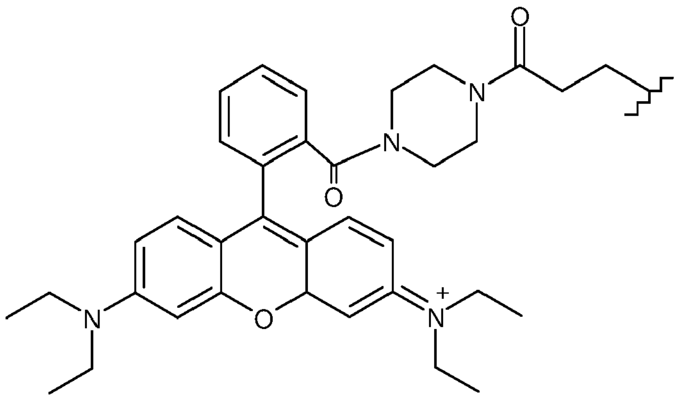

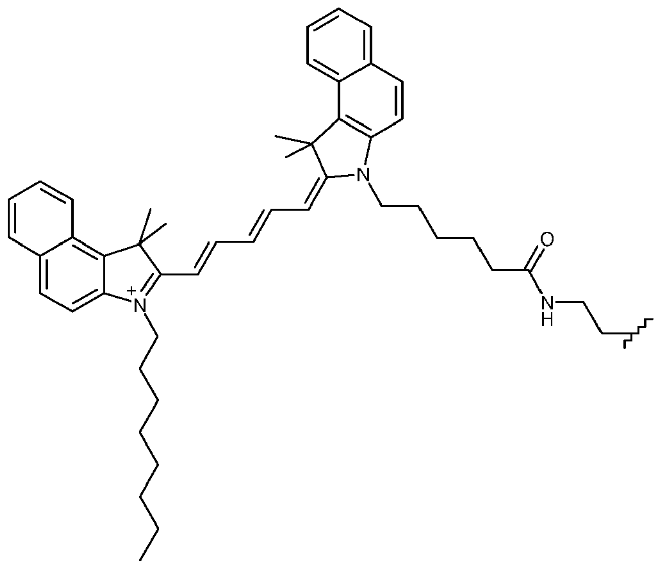

As an example, a BODIPY fluorophore can be chosen among :

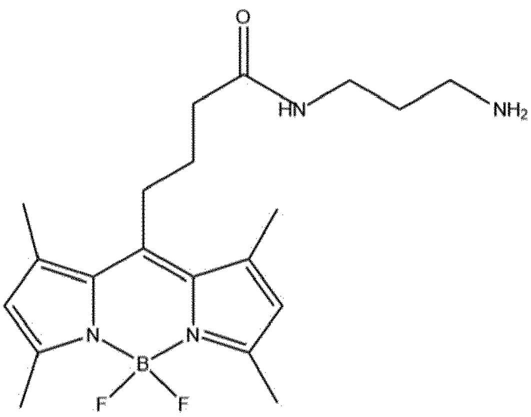

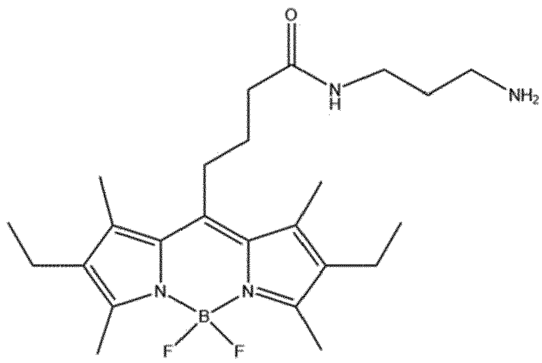

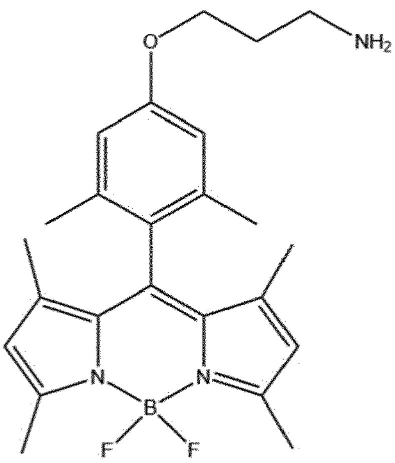

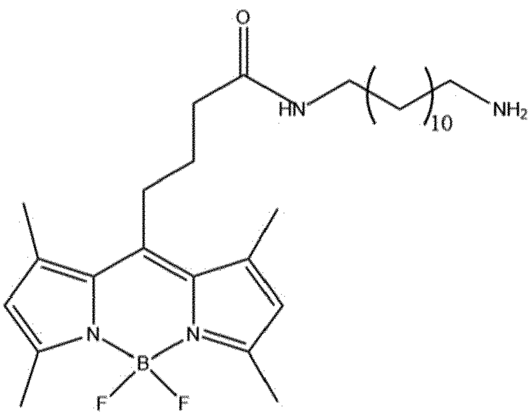

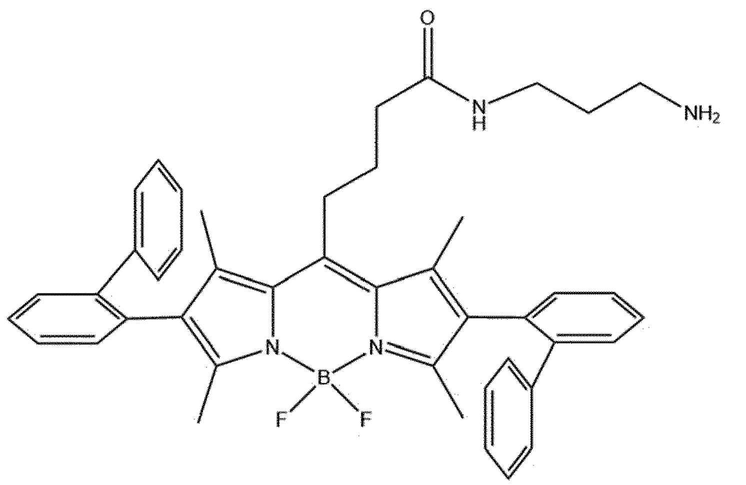

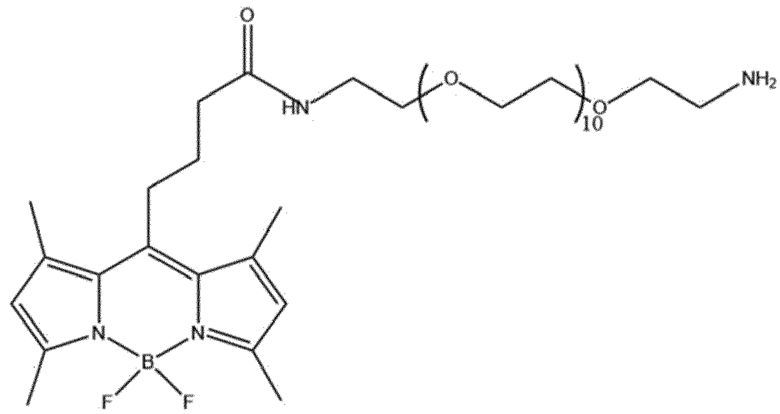

BODIPY fluorophores structures are also illustrated in Figures 3 and 5.

In the above BODIPY fluorophores structures, the terminal amine left a hydrogen atom to give said -NH-Rc group in the structure of the polymeric molecule.

In one embodiment, the fluorophore moiety is located in the core using a hydrophobic linker. In one embodiment, the fluorophore moiety is located in the shell using a hydrophilic PEG linker. In one embodiment, the fluorophore moiety is located at the interface of the core and the shell using a short linker (from 1 to 5 carbon atoms, preferably from 1 to 3 carbon atoms).

An example of preferred linkers are:

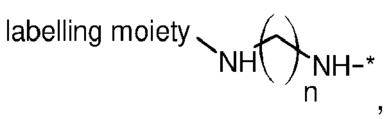,

wherein n is ranging from 1 to 5, preferably from 1 to 3, wherein * represents a covalent bond to the C=O in said structure of the polymeric molecule and wherein "labelling moiety" represents a labelling moiety for example selected from the group of contrast agents, for example Magnetic Resonance Imaging (MRI), X-Ray or phonoacoustic contrast agents.

An example of preferred linkers are:

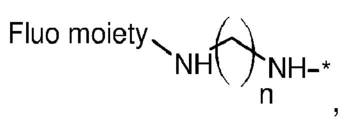,

wherein n is ranging from 1 to 5, preferably from 1 to 3, wherein * represents a covalent bond to the C=O in said structure of the polymeric molecule and and wherein "fluo moiety" represents a fluorophore moiety.

In one embodiment, A in the fluorophore moiety comprises from 2 to 10 carbon atoms.

For example, the fluorophore moiety is a cationic fluorophore moiety, preferentially rhodamine or a cyanine, with a counter-ion.

In one embodiment, said counter ion comprises a borate ion ($B^-$).

More particularly, the counter-ion is chosen among tetraphenylborate ions, preferably among fluorinated tetraphenylborate ions.

In a particular embodiment, said counter-ion is selected from the group consisting of:

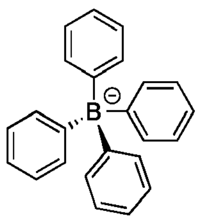 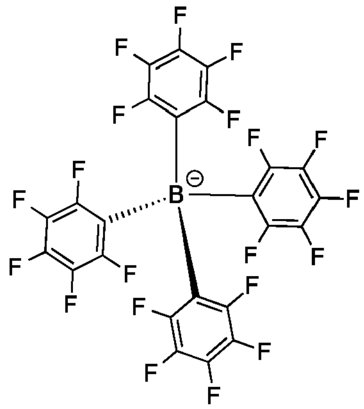 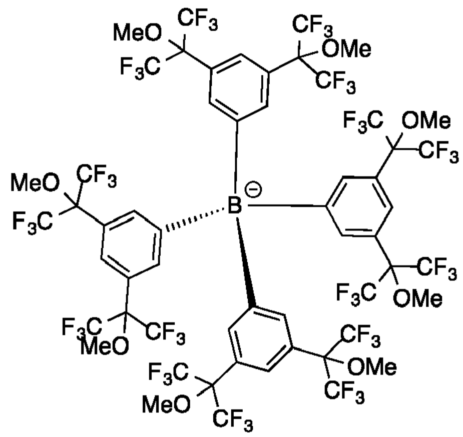

In one embodiment, the counter-ion is in an amount ranging from 1 to 5, preferably from 2 to 3 equivalents, regarding the amount of the cationic fluorophore moiety.

In one embodiment, the percentage of the radicals Rc is a fluorophore ranging from 1% to 50%, preferably from 10% to 50%, based on the total number of the radicals Ra and Rb in said compound of formula (A).

In one embodiment, the radicals Rc, independently of each other, are:

- a labelling moiety, preferably a fluorophore moiety or
- has the following formula (C):

  -X-Y (C)

  wherein

  - X has one of the following formulae: or

  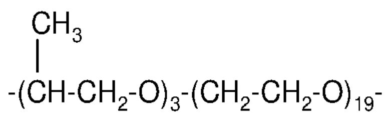

  and
  - Y is $-NH_2$ or $-CH_3$

  wherein the percentage of the radicals Rc being of formula (C) is comprised between 5% and 100%, preferably between 40% and 100%, based on the total number of the radicals Ra and Rb in said compound of formula (A).

In one embodiment, the nanoparticles have a mean diameter of less than 35 nm, preferably comprised between 5 nm and 25 nm, more preferably between 10 nm and 20 nm.

Typically, said nanoparticles are spherical particles. It may be appreciated by Transmission Electron Microscopy (TEM).

For example: 5 µL of the nanoparticle solution were deposited onto carbon-coated copper-rhodium electron microscopy grids that were used following amylamine glow-discharge. The grids were then treated for 1 min with a 2 % uranyl acetate solution for staining and observed with a Philips CM120 transmission electron microscope equipped with a $LaB_6$ filament and operating at 100 kV. Areas covered with NPs of interest were recorded on a Peltier cooled CCD camera (Model 794, Gatan, Pleasanton, CA). Image analysis was performed using the imageJ software.

In one embodiment, the nanoparticles have a mean diameter of less than 15 nm, preferably comprised between 5 nm and 15 nm, more preferably between 10 nm and 20 nm.

Such size or dimension or diameter may be measured by dynamic light scattering (typically measured three times from three independent formulation in milliQ water at 25°C using a Malvern Zetasizer Nano ZSP (Malvern, U.K.); DLS laser was 633 nm.)

Such size or dimension or diameter may be measured by TEM.

Such size or dimension or diameter may be measured by fluorescence correlation spectroscopy (FCS):

For Fluorescence Correlation Spectroscopy: The concentration of polymer was adjusted to 1 μM Bodipy using 80,000 $M^{-1}$ $cm^{-1}$ as molar extinction coefficient value, therefore providing a polymer concentration of 142 nM (7 fluorophores per polymer). The samples were placed in a 96 well plate and were measured with a FCS microscope. Excitation wavelength was 488 nm (power: XXX mW). Fluorescein (50 nM in 0.1 M NaOH) was used as a reference of size (1 nm) and brightness. The data were processed with PyCorrFit.

In one embodiment, the dye or fluorescent moiety concentration in the NP is ranging from 10 mol% to 60 mol %, for example from 20 mol % to 50 mol%

In one embodiment, the nanoparticles according to the invention have a zeta potential (ζ) is higher than -80 mV, ad preferably higher than -50 mV and preferably higher than -20 mV. "Higher" means here that the zeta potential is closer to 0 mV than the given zeta potential.

The present invention also relates to a method for preparing the nanoparticles of the present invention.

In one embodiment, said particles are prepared by reacting compound (I) comprising anhydride functions, said compound (I) having the following structure:

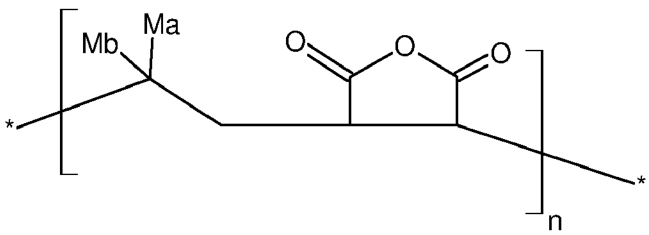

(I),

wherein Ma, Mb and n are as defined in the present invention for formula (A), including any embodiment and preferred features, and

with one or more compounds having the following formula (II):

$NH_2$ - Rc (II)

wherein Rc is as defined according to the present invention, including any embodiment and preferred features, under conditions wherein $NH_2$ group of compound(s) (II) reacts with the anhydride of compound (I), thereby giving a compound of formula (A).

In one embodiment, said particles are prepared by reacting compound (Ia) comprising anhydride functions, said compound (I) having the following structure:

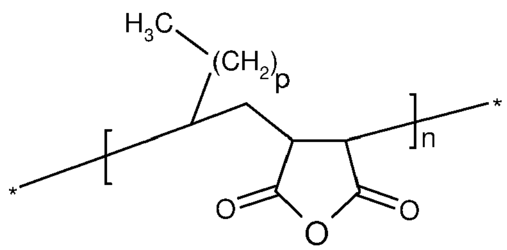

(Ia),

with one or more compounds having the following formula (II):

$NH_2$ - Rc (II)

under conditions wherein $NH_2$ group of compound(s) (II) reacts with the anhydride of compound (Ia), thereby giving a

compound of formula (A).

In one embodiment, said method of preparation of a polymeric nanoparticle, comprising the steps of:

a) contacting poly(maleic anhydride-alt-1-alkylene) with a compound having the following formula (I):

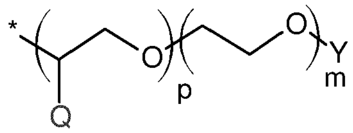

with p, m, Y and Q being as defined in the present invention ;
and with a targeting compound or a labeling compound in order to obtain a compound having the formula (A) as defined in the present invention;
b) isolating said compound of formula (A); and
c) adding said compound of formula (A) to an aqueous medium in order to obtain a polymeric nanoparticle.

In one embodiment, said method comprises reacting a compound of formula (A) wherein Ra and/or Rb represent OH with $NH_2$-Rc said compound(s) (II) in the presence of an activating agent for forming an amide function (N-acylation), such as for example HATU (1-[Bis(dimethylamino)methylene]-1H-1,2,3-triazolo[4,5-b]pyridinium 3-oxid hexafluorophosphate ; Hexafluorophosphate Azabenzotriazole Tetramethyl Uronium). HATU is typically used in the presence of N,N-diisopropylethylamine (DIPEA).

In one embodiment, said aqueous medium is miliQ water, buffered water (Phosphate buffer) or PBS. An example of suitable pH is from 5 to 7.4.

Methods to prepare a compound of formula (I), including compounds of formula (Ia), are known in the art. In particular, method to prepare PMAO are known in the art. Such method could be radical polymerization, for example by using a polymerization initiator which generate a radical at both terminal ends. For example, such polymerization initiators and generated radicals are as follows:

| Initiator | Radical Generated |
|---|---|
| Benzoyl peroxide (BPO) 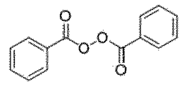 | 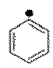 |
| Di-*tert*-butyl peroxide (DTBP) 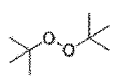 | 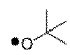 |
| Azobisisobutyronitrile (AIBN) 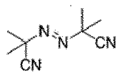 | 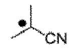 |

Surprisingly, the polymeric molecules according to the present invention are capable of self-folding in aqueous media and form the monomolecular NPs as defined in the present invention.

Polymer self-folding is more prone to provide monomolecular NPs with increasing number of radicals Rc comprising a polyether (PEG).

Preferably, nanoparticles according to the present invention are stable.

The present invention also relates to an aqueous composition comprising:

- at least one polymeric nanoparticle according to the present invention; and
- an aqueous medium, which include but not limited to water, including miliQ water, dionized water, distilled water, buffered water, salted water like PBS.

In one embodiment, said composition is an injectable composition.

Surprisingly, the nanoparticles according to the present invention have a good brightness when bearing a fluorescent moiety. It is preferred to have the fluorescent moiety at the interface of the core/shell to enhance brightness. It is preferred to have a fluorescent moiety bearing a group preventing H-aggregation, for example a bulky group.

In one embodiment, the nanoparticles according to the present invention target a specific target, such as for example a membrane receptor or for example cancer cells, such as for example integrin receptor.

The nanoparticles according to the present invention have few or non-specific interaction so that such NPs are stealth particles. Such stealth particles can for example circulate in the body for extended periods of time without being rejected by the body's immune system. Such stealth property may be assessed by electrophoresis.

In one embodiment, the nanoparticles according to the present invention are drug delivery systems.

In one embodiment, the present invention relates to a pharmaceutical composition comprising one or more type of nanoparticles according to the invention.

In one embodiment, the present invention relates to nanoparticles according of the invention for use in a pharmaceutical composition or for preparing a pharmaceutical composition

Accordingly, the present invention also relates to a pharmaceutical composition, said composition comprising at least one polymeric nanoparticle according to to the present invention.

The present invention also relates to a composition for use in a method of diagnosis, said composition comprising at least one polymeric nanoparticle according to to the present invention.

In one embodiment, the present invention relates to nanoparticles according of the invention or to a pharmaceutical composition comprising one or more type of nanoparticles according of the invention for use in a method of therapeutic treatment.

In one embodiment, the present invention relates to nanoparticles according of the invention or to a pharmaceutical composition comprising one or more type of nanoparticles according of the invention for use in a method of treatment of a cancer, preferably by targeting cancer cells.

In one embodiment, the present invention relates to nanoparticles according of the invention or to a composition comprising one or more type of nanoparticles according of the invention for use in a method of diagnosis.

In one embodiment, the present invention relates to nanoparticles according of the invention or to a pharmaceutical composition comprising one or more type of nanoparticles according of the invention for use in a method of bioimaging, for example cell imaging, typically fluorescence imaging or cell fluorescence.

The present invention also relates to a method of therapeutic treatment or bioimaging comprising the administration of nanoparticles according of the invention or of a pharmaceutical composition comprising one or more type of nanoparticles according of the invention. In one embodiment, nanoparticles or a composition is administered to a human or animal in need thereof. In one embodiment, nanoparticles or a composition is administered in vitro.

In one embodiment, the present invention relates to nanoparticles according of the invention or to a pharmaceutical composition comprising one or more type of nanoparticles according of the invention for use in a method of diagnostic, *in vitro* or *in vivo.*

The present invention also relates to the use of at least one polymeric nanoparticle according to the present invention, as a nanoprobe or as a drug delivery system.

The present invention also relates to the use of at least one polymeric nanoparticle according to the present invention, in bioimaging, cellular imaging or medical imaging.

In the present text, the expression "comprised between X and Y" includes X and Y, unless contrary indication.

In the present text, aspects, embodiments, preferred and advantageous features are combinable according to any possible combinations thereof, unless technical impossible.

**Figure 1.** Synthesis of fluorescent amphiphilic polymers and their resulting NPs after formulation in water. a. BDP1, DIEA, DMF 60°C, then water. b. BDP1, DIEA, J-1000, DMF 60°C; c. BDP1, DIEA, J-1000, HATU, DMF 60°C.

**Figure 2.** TEM images of non-PEGylated NPs (A) and fully-PEGylated NPs (B), scale bar is 150 nm and 30 nm respectively. Normalized absorption (C) and emission (D) spectra of the 3 different NPs (2.5 μM based on dye). (E) Agarose gel (0.5 %) electrophoresis of non-PEGylated, half-PEGylated and fully-PEGylated NPs in water (a, b and c respectively) and in the presence of 5 μM BSA (d, e and f respectively), 0.2 μg per well. The white arrow indicates the level of the wells. Scale bar is 0.5 cm. Laser scanning confocal microscopy images of KB cells incubated for 1h in opti-MEM and in the presence of NPs: non-PEGylated (F), half-PEGylated (G) and fully-PEGylated (H). The concentration of polymer was adjusted in order to have 1 μM dye concentration. Inset in (F) is a 3D reconstruction of a cell containing the polymer. The nucleus (gray color) was stained with Hoechst (5 μg/mL). Excitation wavelength was 488 nm and emission was collected from 498 to 560 nm. The gain of the green channel's PMT was increased from 600 V for F to to 1000 V for G and H. Scale bar is 20 μm.

**Figure 3.** Synthesis of NPs displaying the fluorophores at different locations by means of linkers with various length and hydrophobicity.

**Figure 4.** Characterization of NPs displaying the fluorophores: at the interface, the core and the PEG shell. Normalized absorption (A-C) and emission (D-F) spectra of NPs with increasing dye loading. Evolution of the quantum yield (G), brightness (H) and size (I) of the NPs with increasing dye loading. The brightness was calculated considering a monomolecular NPs and a $\varepsilon$ value of the dye of 80,000 $M^{-1}.cm^{-1}$.

**Figure 5.** Synthesized amino-BODIPYs with various bulkiness patterns.

**Figure 6.** Characterization of NPs functionalized with BDP2, BDP3 and BDP4. Normalized absorption (A-C) and emission (D-F) spectra of NPs with increasing dye functionalization level. Evolution of quantum yield values (G), brightness (H) and size (I) of the NPs with increasing dye functionalization level. The brightness was calculated considering a monomolecular NP and a ε value of the dye of 80,000 M-1 cm-1.

**Figure 7.** (A) Agarose gel (0.5 %) electrophoresis of NPs with increasing BDP4 loading (0.2 μg per well). The fluorescence intensity is color-coded as displayed. The white arrow indicates the level of the wells. Scale bar is 0.5 cm. Single particle fluorescence microscopy images (λ Ex= 532 nm, power density: 3 W cm-2. Integration time was 20×100 ms) of QDots-585 streptavidin conjugate (B) and NPs containing 50% BDP4 (C). Scale bar is 5 μm. 3D Single particle fluorescence microscopy images of QDots-585 (D) and 50% BDP4 NPs (E).

**Figure 8.** Comparison of intracellular distribution of BDP1-based NPs with that of a fluorescent protein (mCherry) and quantum dots (QDots-655). Co-injection in HeLa cells of NPs (20% BDP1, 0.33 μM) with mCherry (1.75 μM) (A, B, C) or with QDots-655 Streptavidin Conjugate (0.05 μM + BSA 5 mg/mL) (E, F and G). NPs are in green (A, E), mCherry and Qdots-655 (QDs) are in red (B and F respectively). C and G are the merge of A and B and E and F respectively with the brightfield channel. D and H are the plots of normalized intensity corresponding to the line in C and G respectively. Scale bar is 10 μm.

**Figure 9.** NMR spectra of PMAO reacted with increasing amount of Jeffamine-1000 (400 MHz, CDCl3). 0% Jeffamine is PMAO polymer with no modification.

**Figure 10.** Size of NPs obtained from polymers with increasing PEGylation rate. The sizes were assessed by DLS measurements of three independent formulations. For 0% PEG loading, non-modified PMAO was directly used.

**Figure 11.** NMR spectra of fluorescent PMAO polymers reacted with increasing amount of Jeffamine-1000 (400 MHz, CDCl3).

**Figure 12.** Fluorescence spectra (A) and normalized spectra (B) of Nile Red (200 nM) at increasing concentration of BDP1 NPs (5 mol%). Concentration of polymer is used. (C,D) Corresponding changes in the fluorescence intensity at the maximum (C) and position of the maximum (D). Excitation wavelength was 540 nm.

**Figure 13.** Schematic view of an embodiment of a nanoparticle of the invention (left) and schematic details of the unfolded/folded polymeric molecule (right) with an example of a labelling moiety located at the interface of the core/shell structure.

## Examples

### Example 1 - preparation of NPs

All starting materials for synthesis were purchased from Alfa Aesar, Sigma Aldrich or TCI Europe and used as received unless stated otherwise. PMAO was purchased from sigma Aldrich, Jeffamine® was kindly offered by Huntsman International. NMR spectra were recorded on a Bruker Avance III 400 MHz spectrometer. Mass spectra were obtained using an Agilent Q-TOF 6520 mass spectrometer. Protocol of synthesis of all new compounds as well as NMR and mass spectra can be found in the supporting information.

Regarding the formulation of the nanoparticles, in an eppendorf was placed 50 microL (100 micro g of polymer) of stock solution of amphiphilic polymer in dioxane at a concentration of 2 $mg.mL^{-1}$. 950 μL of miliQ water or PBS was quickly added and the solution was vigorously mixed with help of pipetting followed by vortexing for 5 s. The final concentration of polymer is 0.1 $mg.mL^{-1}$.

Example 2 - Effect of polyether group (example with PEGvlation)

PMAO is an alternating copolymer composed of maleic anhydrides (Figure 1) that readily react with amines to form stable amides. Primarily, the PMAO's reactivity and the effect of PEGylation level on the size of NPs assembled in water were investigated. To this endeavor, PMAO was reacted with an increasing amount (from 0 to 100 mol% with respect to reactive anhydrides) of Jeffamine® M-1000 (J-1000), a primary amine-terminated copolymer of propylene oxide (PO) and ethylene oxide (EO) with a PO/EO ratio of 3/19 ($H_2N-(PO)_3-(EO)_{19}-OMe$). While $(EO)_{19}$ part ensures PEGylation, short $(PO)_3$ part may help to consolidate the hydrophobic part of future NPs. Poly(propylene oxide) fragment is present as a hydrophobic block in different Pluronic® surfactants, which are extensively used in biomedical applications. The obtained polymers were analyzed by $^1H$ NMR which showed that the amount of PEG chains compared to the fixed amount of aliphatic chains was increasing according to the used feed ratio, thus demonstrating the reliability of the chemistry performed on PMAO (Figure 9). The polymers were dissolved in dioxane at a concentration of 2 mg/mL and then diluted in water to obtain NPs (100 μg/mL). The size of the formed NPs was assessed by DLS measurements. Although the increasing fraction of PEG provides polymers of higher molecular weight, small NPs (< 12 nm) were obtained starting from 20-50% PEGylation (Figure 10). The hypothesis was that the PEGylation decreased the hydrophobic nature of the amphiphilic polymer and therefore in water the PEGylated polymer molecules do not assemble together but fold into monomolecular (single-chain) micelles with a hydrophobic core of long hydrocarbon chains and a shell composed of PEG.

To confirm this hypothesis, three PMAO-based polymers, labeled with 5 mol% of an amino-BODIPY (BDP1), were synthesized: i) A non-PEGylated polymer where all the anhydride functions were hydrolyzed thus providing charged carboxylate groups, ii) A half-PEGylated one where all the anhydride functions were reacted with J-1000, thus still displaying carboxylate groups at the interface and iii) a fully-PEGylated polymer where all the anhydride functions were converted into diamides by condensing PMAO with J-1000 then using a coupling agent to react J-1000 with the formed carboxylate groups (Figure 1). The resulting polymers were purified by exclusion phase chromatography and analyzed by $^1H$-NMR (Figure 11). For the half-PEGylated and the fully-PEGylated polymer, the PEGylation yields, determined by 1H NMR, were 96 and 88%, respectively.

The polymers were dissolved in dioxane (2 mg/mL) and then diluted in water to induce assembly of NPs. The size of formed NPs was first measured by dynamic light scattering (DLS, Table 1). Although the non-PEGylated polymer gave NPs of 33.6 ± 2.4 nm ; the half-PEGylation led to ultra-small NPs of 9.7 ± 0.8 nm. Interestingly, the fully-PEGylated polymer provided slightly larger NPs of 11.5 ± 0.4 nm, probably due to an increased hydrodynamic diameter provoked by the denser PEG shell. According to ζ potential measurements (Table 1), the higher PEGylation level the lower negative charge displayed, clearly because the carboxylate groups were transformed into neutral amides. Indeed, whereas the non-PEGylated NPs displayed high ζ value of -51.6 mV, the half-PEGylation decreased it to - 11.1 mV. The full PEGylation further reduced the zeta potential to a nearly neutral value (-2.7 ± 1.9).

**Table 1.** Physicochemical properties of the PMAO NPs.[a]

| NPs | Size (nm) | PDI | ζ (mV) | $\lambda_{Abs}$ (nm) | FMWH Abs (nm) | $\lambda_{Em}$ (nm) | FMWH Em (nm) | QY (φ) |
|---|---|---|---|---|---|---|---|---|
| **Non-PEGylated** | 33,6 ± 2.4 | 0.23 | -51,6 ± 1.2 | 500 | 22 | 514 | 38 | 0.26 |
| **Half-PEGylated** | 9,7 ± 0.8 | 0.26 | -11,1 ± 2.5 | 499 | 20 | 508 | 27 | 0.72 |
| **Fully-PEGylated** | 11,5 ± 0.4 | 0.45 | -2,7 ± 1.9 | 499 | 20 | 507 | 26 | 0.79 |

[a] For sizes and ζ potential, the data were obtained from triplicates of three different formulation solutions (0.1 mg/mL). For spectroscopic data the polymer concentration was adjusted in order to fix the dye concentration at 2.5 μM, considering a molar extinction coefficient of 80,000 $M^{-1}cm^{-1}$ for a single BODIPY. The quantum yield was measured at 1 μM dye concentration using fluorescein (in 0.1 M NaOH, φ= 0.95) as a reference.

Dynamic Light Scattering and Zeta potential: The size and the surface Zeta potential (z) of the NPs were measured three times from three independent formulations in milliQ water at 25°C using a Malvern Zetasizer Nano ZSP (Malvern, U.K.). DLS laser was 633 nm.

Consecutively, the NPs were imaged by transmission emission microscopy (Figure 2A and 2B). Regarding Spectroscopy, the water used for spectroscopy was Milli-Q water (Millipore), all the solvents were spectro grade. Absorption and emission spectra were recorded on a Cary 400 Scan ultraviolet-visible spectrophotometer (Varian) and a FluoroMax-4 spectrofluorometer (Horiba Jobin Yvon) equipped with a thermostated cell compartment, respectively. For

standard recording of fluorescence spectra, the emission was collected 10 nm after the excitation wavelength. All the spectra were corrected from wavelength-dependent response of the detector. Quantum yields were determined at optical density ≤0.1 by comparison with a reference according to their excitation and emission wavelengths: Fluorescein in (0.1 M NaOH, $\phi$= 0.95) or Rhodamine 6G in water ($\phi$= 0.95).

The non-PEGylated NPs displayed a round shape with sizes similar to those measured by DLS (32.4 ± 5.2). Remarkably, fully-PEGylated NPs revealed ultrasmall round-shaped nanostructures of quite narrow distribution (9.8 ± 0.8 nm). These images tend to indicate that PEGylation in PMAO leads to formation of particles containing a single polymer molecule or only few of them. The round shape and small size of fully-PEGylated NPs also suggest that the polymers are folded into spherical (globular) structures rather than present in form of out-stretched polymer brush. Theoretically, the latter form would be 42-70 nm long considering all-*trans* configuration of the hydrocarbon backbone of PEGylated PMAO with molecular weight 30,000-50,000 Da (85-140 repetitive units).

Spectroscopic studies allowed to evaluate the number of polymer chains per particle. Upon PEGylation, convergent signs of de-aggregation of the dyes were observed: i) The blue shifted shoulder (at ~470 nm), which is a typical sign *H*-aggregates, decreased (Figure 2C), ii) the emission spectra shifted to the blue (7 nm between non- and fully-PEGylated NPs) accompanied by a decrease in the broadening (see FMWH values in Table 1 and Figure 2D), and finally iii) the measured quantum yields were significantly increased from 0.26 to 0.79 (Table 1). These observations suggest that high PEGylation level prevents form the formation of oligo-molecular NPs and thus favor the formation of monomolecular NPs where the fluorophores are better separated (Figure 1).

The inventors also verified whether incorporation of BDP1 into the polymer affected BDP1 dye properties, which was done by comparing absorption and emission spectra of BDP1-amine (BDP1) and labelled fully-PEGylated polymer (5 mol% of BDP1) in organic solvents and water . In apolar dioxane and more polar PEG4, absorption and emission spectra of BDP1-polymer were very similar to those of free dye BDP4-$NH_2$, indicating that grafting to the polymer does not modify spectroscopic properties of the dye in solubilized form. Moreover, there was practically no spectral difference between these two organic solvents. However, in water, the absorption spectrum of the free dye (BPD1-$NH_2$) was blue shifted compared to organic solvents, and its emission spectrum was broadened, whereas the labelled polymer in water showed virtually the same spectral profile as in organic solvents. The conclusion is that in water, the labelled polymer adapts a compact form that shields BDP1 dye from bulk water.

Next, the NPs were analyzed by electrophoresis. The electrophoresis was performed on an agarose gel (0.5%) in TAE buffer (TRIS, acetate, EDTA). In each well, 20 μL of a solution of NPs (0.01 mg/mL in water) were cautiously added (0.2 μg polymer per well). The gel was gently covered with TAE then a tension of 125 V was applied for one hour. The gels were visualized with an ImageQuant LAS 4000 (GE Healthcare Life Sciences) using Cy2 (473 nm) or Cy3 (532 nm) excitation channels and by acquiring 10 images with an exposition time of 10 seconds. The images were treated with ImageJ.

Owing to the high density of negative charges, the non-PEGylated NPs migrated fast to the anode, whereas the PEGylated NPs moved slower and displayed clear spots denoting their high homogeneity (Figure 2E). In the presence of BSA the non-PEGylated NPs migrated as a smear and reduced their migration towards the anode, probably because they interacted non-specifically with BSA. In sharp contrast, the PEGylated NPs did not change their migration profile when incubated in the presence of BSA, showing the absence of non-specific interactions. As another illustration of the PEGylation effect, the NPs were incubated for 1h in the presence of KB cells before being washed out (Figure 2F-H). The non-PEGylated NPs showed clear non-specific binding to the cells and aggregation due to precipitation in the high ionic strength medium (Opti-MEM). On the other hand, the cells incubated with PEGylated NPs did not display any fluorescence signal thus demonstrating the protecting effect of the PEG shell against non-specific interactions. In accordance with these results, cytotoxicity assays using MTT test revealed that unlike non-PEGylated polymer, PEGylated polymers did not affect the cells viability at concentrations up to 50 μg/mL. Cytotoxicity assay of the NPs was quantified by the MTT assay (3-(4,5-dimethylthiazol-2-yl)-2,5-diphenyltetrazolium bromide). A total of $1x10^4$ HeLa (ATCC® CCL-2™) cells/well were seeded in a 96-well plate 24 h prior to the cytotoxicity assay in Dulbecco's Modified Eagle Medium (Gibco Lifetechnologies -DMEM) complemented with 10% fetal bovine serum, Gentamicin (100 μg/mL), L-Glutamine (2 mM), non-essential amino acids (1 mM), MEM vitamin solution (1%) and were incubated in a 5% $CO_2$ incubator at 37°C. After medium removal, an amount of 100 μL DMEM containing NPs at various concentrations (10 to 50 μg polymer/mL) was added to the cells and incubated for 24 h at 37°C (5% $CO_2$). As control, for each 96-well plate, the cells were incubated with DMEM containing the same percentage of dioxane as the solution with the tested NPs or with triton X-100 0.1% as a positive control of cytotoxicity. After 24h of a dye incubation, the medium was replaced by 100 μL of a mixture containing DMEM and MTT solution (diluted in PBS beforehand) and the cells were incubated for 4 h at 37°C. Then, 75 μL of the mixture was replaced by 50 μL of DMSO (100%) and gently shaken for 15 min at room temperature in order to dissolve the insoluble purple formazan reduced in the living cells. The absorbance at 540 nm was measured (absorbance of the NPs at 540 nm were taken into account). Each concentration of dye was tested in sextuplicate in 3 independent assays. For each concentration, the percentage of cell viability was calculed in reference of the control DMEM and dioxane.

Example 3 - Measure of monomolecular nature of PEGylated polymer based NPs (example with PMAO):

FCS is a powerful spectroscopic technique providing simultaneously information on size, concentration, and brightness of fluorescent nanomaterials, including polymeric NPs. We used FCS to assess the behavior and composition of our NPs in water, PBS and PBS containing 10 μM BSA (Table 2). FCS measurements were performed on a home-built confocal set-up based on a Nikon inverted microscope with a Nikon 60x 1.2NA water immersion objective. Excitation was provided by a cw laser diode (488 nm, Oxxius). For the fluorescence signal detection, a fibered Avalanche Photodiode (APD, PerkinElmer, Fremont, CA) connected to an on-line hardware correlator (ALV7000-USB, ALV GmbH, Germany) was used. Before the APD, the fluorescence signal was filtered through a 405/488/ 532/635 nm BrightLines quad-edge laser-grade dichroic (Semrock, NY) and ET525/50 single band-pass filter (Chroma Corp., Rockingham, VT). The fluorescence signal was further processed by an ALV7002/USB digital correlator (ALV, Langen, Germany). Typical acquisition time was 5 min (10-30 scans) with an excitation power of 0.5 mW at the laser output level.

The data were analyzed using the PyCorrFit software. Assuming that NPs diffuse freely in a Gaussian excitation volume and taking into account triplet blinking, the correlation function, G(τ), calculated from the fluorescence fluctuations was fitted according to equation (1):

$$G(\tau) = \frac{1}{N}\ \frac{(1-F+Fe^{\frac{\tau}{\tau F}})}{(1-F)}\ (1+\frac{\tau}{\tau d})^{-1}\ (1+\frac{1}{S^2}\frac{\tau}{\tau d})^{-1/2}\quad (1)$$

where $\tau_d$ is the diffusion time, $N$ is the mean number of fluorescent species within the excitation volume, and $S$ is the ratio between the axial and lateral radii of the excitation volume; $F$ is the fraction of emissive species that have entered the triplet state and $\tau_F$ is the corresponding triplet state relaxation time. The measurements were done with respect to a reference dye fluorescein (FL) (Sigma-Aldrich) at 50 nM concentration in 0.1 mM NaOH. The hydrodynamic diameter, d, of NPs was calculated as $d_{NPs} = \tau_{d(NPs)}/\tau_{d(FL)} \times d_{FL}$, where $d_{FL}$ is the hydrodynamic diameter of fluorescein (1.0 nm). The concentration of NPs was calculated from the number of species by $C_{NPs} = N_{NPs}/N_{FL} \times C_{FL}$, where $N_{NPs}$ and $N_{FL}$ are number of emissive species of NPs and fluorescein, respectively, and $C_{FL}$ is fluorescein concentration (50 nM).

200 μL of each samples were placed in a 96-well plate. Generally, FCS measurements were done 30 min after dilution of NPs in the corresponding medium (water, PBS or PBS with BSA). The concentration of polymer was adjusted to 1 μM BODIPY using 80,000 $M^{-1}$ $cm^{-1}$ as molar extinction coefficient value, therefore providing a polymer concentration of 140 nM (7 fluorophores per polymer).

In water, the measured sizes were consistent with those obtained by DLS, namely 34, 8.6 and 11.4 nm for non-PEGylated, half-PEGylated and fully-PEGylated NPs respectively. In PBS, non-PEGylated NPs formed large aggregates (-7-fold larger than in water) due to their hydrophobic and charged nature. Interestingly, although the half-PEGylated NPs increased their size and decreased their concentration in PBS and in the presence of BSA, denoting a slight aggregation of the polymers molecules due to non-specific interactions, the fully-PEGylated NPs were negligibly affected (Table 2). This suggests that a high degree of PEGylation is necessary to lead to stealth and stable NPs with highly reduced non-specific interactions with proteins, which is in line with studies on other types of NPs. The obtained result is also in good agreement with the electrophoresis data, where the effect of BSA was not observed in PEGylated NPs.

As hypothesized previously, high PEGylation level might tend to form NPs composed of a small number of polymer molecules and even monomolecular NPs. As a first hint, a theoretical calculation based on i) the molecular weight of the fully-PEGylated PMAO polymers (~300,000 $g.mol^{-1}$), ii) the assumption that the formed NPs are spherical and iii) the assumption that the polymers have a density of ~1000 $Kg/m^3$, provided a theoretical size of 10.1 nm for a monomolecular NP. This value matches well the measured sizes of fully-PEGylated PMAO obtained by 3 different technics (DLS, TEM, FCS), supporting also organization of the polymer in the folded globular form (Figure 1). Second, FCS is perfectly adapted to confirm this hypothesis as it can measure the concentration of NPs. When monomolecular NPs are obtained their concentration should be the same as the concentration of the polymer. The concentration of fluorophore was set at 1 μM by absorption spectroscopy. Since, on average, 7 dyes are displayed on a single polymer (5% of 140 units per polymer), the estimated concentration of single polymer molecules was 140 nM (1 μM divided by 7). For the PEGylated NPs, the concentration in water measured by FCS perfectly matched the estimated concentration of polymer molecules (Table 2), thus confirming our hypothesis on monomolecular nature of PEGylated PMAO based NPs.

**Table 2.** Physicochemical properties of the PMAO NPs obtained by FCS measurements.

| Polymer | Conditions | Size (nm)[b] | [Particules] (nM) | Number of polymer per NP[c] | Relative brightness[d] |
|---|---|---|---|---|---|
| **Non-** | Water | 34.0 | 5.79 | 24.5 | 22.8 |

(continued)

| Polymer | Conditions | Size (nm)[b] | [Particules] (nM) | Number of polymer per NP[c] | Relative brightness[d] |
|---|---|---|---|---|---|
| PEGylated | PBS | 261 | 0.133 | 1070 | 320 |
| | 10 μM BSA[a] | 221 | 0.128 | 1110 | 413 |
| Half-PEGylated | Water | 8.6 | 143 | 0.994 | 4.00 |
| | PBS | 11.5 | 60.2 | 2.36 | 5.23 |
| | 10 μM BSA[a] | 11.9 | 81.3 | 1.75 | 4.73 |
| Fully-PEGylated | Water | 11.4 | 138 | 1.03 | 4.98 |
| | PBS | 12.2 | 110 | 1.29 | 4.98 |
| | 10 μM BSA[a] | 13.2 | 116 | 1.23 | 5.05 |

[a] In PBS. [b] Obtained considering the reference (fluorescein) being 1 nm. [c] The polymer concentration was adjusted to 1 μM fluorophore (corresponding to 140 nM polymer concentration). [d] Relative Brightness compared to the reference (fluorescein). The values were corrected according to the molar extinction coefficient values at the excitation wavelength (488 nm).

Example 4 - Measure of brightness

FCS also provided information regarding the brightness of the formed NPs. Fluorescein was used as a reference (50 nM in NaOH 0.1 M, ϕ0.95) and the brightness of the NPs was expressed in equivalent number of fluorescein molecules. Even though non-PEGylated NPs in water are composed of 24 polymers (~168 dyes) the brightness was equivalent to 23 fluorescein molecules due to high quenching of the fluorophores within the compacted polymers. On the other hand, half-PEGylated NPs and fully PEGylated NPs, composed of a single polymer, are as bright as 4 and 5 fluorescein molecules, respectively. Thus, the FCS data also confirmed that a high level of PEGylation enhanced the brightness of the obtained NPs.

Example 5 - Characterization of hydrophobic core of NPs

In contrast to the out-stretched configuration of a polymer brush, the folded monomolecular nanoparticle is expected to bear a hydrophobic core (Figure 1), similar to that of micelles made of surfactants. To evidence the presence of this core, we used a solvatochromic dye Nile Red, which is a common tool for identifying low polar environment of micelles[81] and single-chain polymeric NPs. Fluorescence spectra of Nile Red at fixed concentration (200 nM) were recorded with addition of different concentrations of fully PEGylated PMAO polymer with 5 mol% BDP1 (Figure12. A drastic increase in the fluorescence intensity of Nile Red was observed accompanied by ~30 nm blue shift of the emission band (Figure12A,B,D. The increase in the intensity stabilized above 200 nM polymer concentration (Figure12C, which corresponded well to 1/1 dye/polymer molar ratio. Below this concentration, multiple Nile Red molecules per particle can be present, which leads to dye self-quenching, explaining lower intensity at lower polymer concentrations. On the other hand, the blue shifted emission of Nile Red at ~630 nm indicates the presence of a hydrophobic binding site in these NPs. The NPs were compared with micelles of Tween 80, having a hydrophobic core formed by hydrocarbon chains. Similar spectral changes were observed for Nile Red with addition of Tween 80, whereas above CMC (12 μM), blue shifted emission centered ~640 nm was observed. The results suggest that in water the fully PEGylated polymer presents a hydrophobic binding site for a single emissive Nile Red molecule and this site is even less polar than that in Tween 80 micelles, which support our model of the single-chain polymer folding into a particle with a apolar core. Lower polarity inside the NPs is probably related to their polymeric nature that drastically decreases dynamics of its amphiphilic units, in contrast to micelles based on small-molecule surfactants.

Example 6 - effect of the linker

Encouraged by the interesting properties these NPs, we aimed at enhancing their brightness to the maximum. The brightness (B) of a NP composed of several fluorophores is defined by Equation 1 where n is the number of fluorophores that compose the NP, ε is the molar extinction coefficient value of the used fluorophore and ϕ is the measured quantum yield of the NP.

Eq. 1 $$B = n \times \varepsilon \times \phi$$

According to this equation, the brightness of a NP can theoretically be enhanced by increasing the fluorophore loading (n). However, concentrating fluorophores within a small volume tends to provoke *H*-aggregation by $\pi$-stacking of the fluorophores thus leading to the aggregation caused quenching (ACQ) phenomenon that lowers the quantum yield.[24] In order to prevent ACQ, we first investigated on the effect of the fluorophores' localization within the NP as the local concentration of the dye within the latter may vary (Figure 3). For this purpose, various linkers between the polymer and the fluorophore were used: i) a short linker that maintains the fluorophores at the interface ii) a long hydrocarbon chain (C12) that directs the fluorophores into the core of the NP by hydrophobic interactions with the hydrocarbon chains of PMAO and iii) a hydrophilic $PEG_{12}$ that displays the fluorophore in the PEG shell of the NP. In addition to modifying the local dye concentration, these three different locations may alter hydration, polarity or viscosity of the dye environment that should also affect its quantum yield.

PMAO was then reacted with the different amino-BODIPYs (Figure 3) at various loading percentage (from 2 to 50% of reactive sites) and was then fully PEGylated. The obtained polymers were purified and served to formulate the nanoparticles in water. The quenching phenomenon was monitored by the increase of the blue shifted shoulder on the absorption spectra (*H*-aggregation), the broadening of both absorption and fluorescence spectra as well as the decrease of the quantum yield values (Figure 4). The results showed that the contribution of the shoulder in the absorption spectra (Figure 4A-C) as well as the broadening of emission spectra (Figure 4 D-F) were less important when the fluorophores were used with PEG linker (i.e. located in the PEG shell). However, the decrease of quantum yield values upon dye functionalization percentage was fast in all cases and follows the same trend depicting a significant quenching of the dye regardless of the dyes' location (Figure 4G). Consequently, the brightness quickly reached a threshold below 400,000 $M^{-1}.cm^{-1}$ at only 10% loading (Figure 4H). It is noteworthy that when the BODIPYs were concentrated in the core of the NP using a hydrophobic linker (above 30% dye functionalization), the brightness slightly increased while the emission spectra broadened and shifts to the red. This could be attributed to complex forms of aggregation including emissive aggregation forms, such as *J*-aggregates. Importantly, DLS measurements showed that small sizes of NPs were retained (10 to 14 nm) up to 40 % dye functionalization (Figure 4I). Additionally, NPs with 5% dye functionalization were compared by electrophoresis in the absence and presence of BSA.The NPs bearing the fluorophore through a short linker (i.e. dye located at the interface) were less smeary indicating their higher homogeneity. Moreover, in the presence of 5 $\mu$M BSA, these NPs were found to migrate in the same manner, as a single spot, whereas the NPs displaying the fluorophore at the core (with hydrophobic linker) or the PEG shell (PEG linker) started providing extra spots probably attributed to non-specific interactions with BSA. In the light of these results, the short linker was retained for the rest of the study.

Example 7 - Effect of the dyes' bulkiness on the brightness of the NPs

The second approach to prevent aggregation-caused quenching and to improve the brightness of the NPs was to investigate on the BODIPY's bulkiness. Enhancing the bulkiness of fluorophores to prevent aggregation was successfully applied for different dyes, including BODIPY and perylene diimide. To this aim, we synthesized and used three new amino-functionalized BODIPYs with various bulkiness patterns and with a short linker in order to localize the fluorophore at the interface (Figure 5). The bulkiness of the BODIPY was tuned by means of substitution at the *meso* position with a dimethyl phenyl group (BDP2) and at the *beta* positions with ethyl (BDP3) or diphenyl groups (BDP4). Here again, PMAO was reacted with the different amino-BODIPYs at various functionalization percentage and were then fully PEGylated.

First, it is noteworthy that BODIPY dyes grafted to the polymer displayed different photophysical properties. From BDP2 to BDP4, longer absorption and emission wavelengths were observed (Figure 6) due to the increasing electron donor effect of the substituents in $\beta$ position. Additionally, broadening of the spectra was observed which was ascribed to supplementary vibrational states due to the $\sigma$-bond rotations of the $\beta$ substituents. Although broad spectra can be detrimental in multicolor imaging, where crosstalk can take place between the imaging channels, the broad absorption spectra of BDP4-based NPs can be an advantage as it can be excited by various excitation sources (e.g. 488 and 532 nm). Further spectroscopic studies showed a dramatic influence of BODIPY's bulkiness on the self-quenching effect. In comparison to BDP1, bulkier BDP2 and BDP4 showed systematically lower increase in the short-wavelength shoulder (Figure 6A-C), and all three new derivatives BDP2-4 displayed narrower emission bands at high dye functionalization level (Figure 6D-F). Remarkably, in the case of the bulkiest fluorophore, BDP4, the spectroscopic changes upon increase in the dye functionalization level were the smallest, showing the important effect of the bulky groups (Figure 6C and F). Even though the use of BDP2 and BDP3 helped in slowing down the quantum yield decrease compared to the initial BDP1, the brightness was limited to 800,000 $M^{-1}$ $cm^{-1}$ with 20% functionalization level using BDP3. The use of BDP4 allowed to maintaining quantum yield values above 0.6 regardless of the dye functionalization level thus leading to impressive brightness values up to ~2,500,000 $M^{-1}$ $cm^{-1}$ per monomolecular NP. We expect that the bulky groups effectively prevent BDP4 dyes from $\pi$-$\pi$ stacking and formation of poorly emissive H-aggregates inside NPs.

The hydrodynamic diameters of the obtained NPs only slightly increased upon loading of the dye up to 14 nm and

therefore allowed us to obtaining small and bright water soluble NPs. FCS measurements for BDP4 NPs (5 mo% loading) confirmed the small size, single-polymer chain nature of NPs and high particle brightness. Moreover, similarly to NPs based on BDP1, BDP4 NPs showed high stability after incubation in PBS and BSA, because their size, concentration and brightness were close to those in water.

In order to check whether the loading of BDP4 could affect the homogeneity of the formed NPs, the latter were analyzed by electrophoresis. The results confirmed the increasing brightness upon dye loading as well as a remarkable homogeneity even at the highest dye loading (Figure 7A). Our brightest NPs were then imaged by single molecule microscopy and were compared to quantum dots that emit in the same wavelength range (QDot-585, streptavidin conjugate). For single-particle microscopy, the following protocol was implemented: Prior to imaging the surface of 8 wells-Lab-Tek chambered coverglass was treated for 1 h with an aqueous solution of KOH (1 M). Then, the wells' surface was extensively rinsed with ultrapure water and incubated with PEI solution (1 mg/mL in tris buffer) at RT for 15 min followed by rinsing with ultrapure water. The solutions of NPs were added and allowed to stay at RT for 30 minutes in the dark before the being removed and replaced by mQ water. Single-particle measurements were performed in the total internal reflection (TIRF) mode using a Nikon Ti-E inverted microscope with a 100× objective (Apo TIRF, oil, NA 1.49, Nikon). The excitation was provided by OXXIUS laser at 532 nm with 3.0 W/cm$^2$ excitation power density. The presented images were an average of the first 20 frames (recorded at a 100 ms integration time. The single-particle analysis was performed using Fiji software, similarly to the previously described protocol. Briefly, particle locations were detected through a Fiji routine applied to a projection (maximum intensity) of 100 frames using an appropriate threshold. The mean intensities of circular regions of interest with a diameter of 5 pixels around the found particle locations were then measured. Background subtraction was then achieved by measuring the mean intensities in circular bands around the circular regions of interest and subtracting them. Finally, integrated intensity in all circular regions of interest was calculated and used for building the histograms of particle intensities. For BDP4 NPs and QDots-585, 150 and 50 particles were analyzed, respectively. Average intensity (± sem) of the two types of NPs was compared.

Based on analysis of the average single particle brightness, the NPs loaded with 50% BDP4 was found to be 5.3 ± 1.3 fold brighter than QDots-585 under the same conditions of illumination, recording and processing (Figure 7B-E). The intensity distribution histograms confirmed the significantly higher brightness of our NPs vs QDots-585, although the size distribution of the former was larger, probably related to the distribution of the PMAO polymer size and the dye grafting ratio. The estimated brightness following equation (1) provided a value of $2.4 \times 10^6$ M$^{-1}$ cm$^{-1}$ for BDP4 NPs at an excitation wavelength at 532 nm. On the other hand, estimated brightness of QDot-585 at the same excitation wavelength is $\varepsilon \times QY = 310\,000$ M$^{-1}$ cm$^{-1}$ $\times 0.67 = 2.1 \times 10^5$ M$^{-1}$ cm$^{-1}$. Consequently, theoretically our NPs should be 11.4 times brighter, which is in line with 5.3-fold difference observed in the single-molecule microscopy. Somewhat lower than expected brightness of BDP4 NPs can be explained by the configuration of filters in the microscope, which collects light less efficiently in case of BDP4 NPs, because its emission band is closer to the excitation wavelength compared to that of QD585. It should be noted that, to keep the same imaging conditions for both types of NPs, QDs were not excited at UV-violet region, where they exhibit significantly higher extinction coefficients. Excitation of QDot-585 in this spectral region (around 400 nm) could result in the comparable brightness to our NPs, although this excitation region is less favorable for bioimaging because of phototoxicity and cell autofluorescence. Next, we aimed at studying the photostability of our NPs. Regarding photostability assay, aqueous solutions of NPs were placed in a 50 μL quartz cuvette and the entire solution was continuously irradiated at 486 nm for 20 min with slit opening at 16 nm. The fluorescence intensity was monitored at the maximum emission wavelength determined by emission spectra prior to kinetic measurements. For fair comparison, the concentrations were adjusted to obtain identical optical density of 0.015 at the excitation wavelength of 486 nm. Emission slit opening was set to obtain optimal signal.

First, we compared NPs at various BDP1 loading and as a standard we used fluorescent polystyrene NPs (FluoroSpheresTM 505/515, ThermoFisher) emitting in the same spectral range. As expected FluoroSpheresTM displayed robust photostability, whereas BDP1-loaded NPs displayed lower stability losing up to 50% of the fluorescence intensity. Remarkably, a decrease in the photostability was observed with increase in the dye loading. This phenomenon might be attributed to intermolecular reactions in the excited state which is promoted by the close proximity of the fluorophores. Similar loss of photostability were observed earlier for polymeric NPs loaded with perylenediimide65 and rhodamine69 dyes. In a second step the BDP4-loaded NPs were studied where a correlation between dye loading and photostability was also observed. However, compared to BDP1, BDP4-loaded NPs were significantly more photostable with similar photostability of our NPs with 20 mol% BDP4 and QDots-585. This photostability improvement might be ascribed to the difference in the bulkiness between BDP4 and BDP1. Indeed, the steric hindrance of the dyes prevents from ACQ and collisions that might promote reaction and then bleaching of the dye. However, at the highest loading (50 mol%), the NPs displayed impressive decay of fluorescence intensity depicting a low photostability. The latter indicates that a compromise should be found between dye loading and photostability to achieve the best performance.

Finally, the inventorswondered if such a high degree of dye functionalization could be detrimental for the cell viability. Therefore, MTT test was performed on NPs loaded with 50% BDP4 and showed no apparent cytotoxicity.

Example 8 - Intracellular behavior of NPs

As demonstrated above, we developed ultrabright NPs with size comparable to large proteins. These NPs and proteins share another common point as they both can display reduced non-specific interaction in biological media. Indeed, proteins are able to circulate freely in the cell until they rich their biological target. Here, we compared the cytosolic behavior of our NPs with the one of proteins. To do so, HeLa cells were co-microinjected with green emitting NPs (20% BDP1 functionalization, 10 nm size, see Figure 4). BDP1 was chosen instead of other BDP to avoid cross talk in the red channel) together with mCherry (3.5 nm size, 28.8 KDa), a red emitting fluorescent protein (Figure 8A-C). Moreover, analogous experiment was done by co-microinjecting the same NPs with far red emitting Qdots-655 as a bright water-soluble nanoparticles standard (Figure 8E-G,). The streptavidin-coated QDots-655 was selected, so that their size (20 nm diameter, according to the manufacturer) is ~2-fold larger compared to the BDP1 NPs. First, the fluorescence microscopy images showed that our NPs (green signal) occupied the whole cell surface denoting a smooth distribution within the cytoplasm with no sign of aggregates. Then, the distribution of the green signal from the NPs of invention with the one of the red signal from the co-injected fluorescent protein or QDots-655 were compared. Remarkably, when the NPs were co-injected with mCherry, their fluorescence signals overlaid in a quite homogeneous manner (Figure 8C) and their fluorescence intensity decrease similarly (down to 60% for mCherry and 50% for NPs) from the injection point to the extremities of the cell (Figure 8D). In sharp contrast, the QDots-655 signal decreased rapidly when it got away from the injection point and the fluorescence intensity decreased down to 20% at the extremities of the cell (Figure 8H). These results suggest that the herein developed fluorescent NPs have similar intracellular spreading compared to a small hydrophilic protein, probably because of their small (10 nm) size and thick PEG shell that protects NPs from non-specific interactions thus insuring them a fast progression in the cytoplasm. Less efficient intracellular spreading of the QDs compared to our NPs is probably related to the larger size of streptavidin-coated QDots-655. This observation is in line with recent works showing that increase in size of NPs in the range 10-40 nm can drastically decrease their intracellular diffusion and spreading in the cytosol. Thus, the ultra-small size and strong PEGylation make our single-chain polymeric NPs compatible with intracellular applications.

Conclusions:

Replacing fluorescent dyes with bright nanoparticles can boost performance of fluorescence sensing and imaging. However, nanoparticles are much larger than molecules and their non-specific interactions and homogeneity is difficult to control. To address this issue, the inventors proposed to explore a biomimetic phenomenon of single-chain polymer folding. In contrast to previously reported fluorescently-labeled water-soluble hydrophilic polymers, the inventors designed fluorescent amphiphilic alternating polymers that fold into globular monomolecular nanoparticle, similarly to proteins. The folded micelle-like globular structures of the polymer is expected to ensure compact uniform structure with alkyl chains in the hydrophobic core and tightly packed polar shell at the surface to prevent non-specific interactions. The design is based on highly PEGylated poly(maleic anhydride-alt-1-octadecene) functionalized with large amount (up to 50 mol%) of BODIPY dyes. The inventors found that strong PEGylation rate is required to ensure folding into the monomolecular particles. Fluorescence correlation spectroscopy suggested that the obtained NPs are stable in physiological salt conditions and do not exhibit non-specific interactions with proteins, probably due to highly dense PEG shell. Then, the linkers of different length and polarity connecting BODIPY to the polymer were tested and the inventors found that short medium-polar linker ensured small size and good fluorescent properties of NPs, which suggest that the particle interface is the most optimal location of the dye. Importantly, incorporation of bulky groups into BODIPY dye prevented it from H-aggregation inside the particle, which allowed us to dramatically enhance its fluorescence quantum yield for high dye grafting ratio. The obtained NPs reached an impressive brightness value of 2,500,000 M-1 cm-1 with a hydrodynamic diameter of 14 nm and were found to be 5-fold brighter than QDot-585 (~20 nm) at 532 nm excitation, according to single-particle fluorescence microscopy. Finally, when microinjected in cells, these small and stealth NPs diffused more evenly inside the cytosol than QDots, highlighting the importance of the size for reaching sterically hindered compartments of the cytosol. Remarkably, the intracellular spreading of our NPs was similar to that of a red fluorescent protein, which shows that they can be considered as artificial protein analogues, compatible with the intracellular applications. The proposed fluorescent monomolecular polymeric NPs is a new nanomaterial with controlled small and uniform size and bright fluorescence for numerous bioimaging applications.

## Claims

1. A monomolecular polymeric nanoparticle (NP) having a core and a shell, wherein said NP consists of a polymeric molecule having a lipophilic unit and a unit comprising a polyether (amphiphilic polymer), said lipophilic unit forming said core and said unit comprising a polyether forming said shell.

**2.** A polymeric nanoparticle (NP) having a core and a shell, wherein said NP comprises or consists of 1 to 10 polymeric molecules having a lipophilic unit and a unit comprising a polyether, said lipophilic unit forming said core and said unit comprising a polyether forming said shell, wherein said polymeric molecule has the following formula (A):

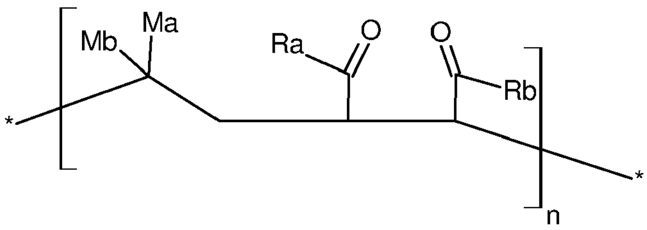

**(A)**

wherein

- n is ranging from 2 to 10,000;
- * represents at each occurrence a radical of a polymerisation initiator;
- Ma and Mb are each independently a hydrogen atom, a, linear or branched, alkyl group having from 1 to 30 carbon atoms, for example from 8 to 30, preferably from 10 to 25, and preferably from 10 to 20, and has for example 16 carbon atoms, or an aryl, for example a benzyl ;Ra and Rb, independently of each other, are OH or a group having the following formula (B):

-NH - Rc **(B)**

wherein, the radicals Rc, independently of each other, are:
- a labelling moiety or a targeting moiety; or
- represent a group of formula (C):

-X-Y (C)

wherein said labelling moiety or said targeting moiety and said group of formula (C) are preferably both present in said polymeric molecule;
wherein

- X comprises a polyether;

and

- Y is selected from the group consisting of: H, OH, $OCH_3$, $CH_3$, $NH_2$, SH, a ligand, and a functional group ($N_3$, alkyne, strained alkyne like DBCO, dienophile, maleimide, able to bind a ligand; and

wherein the percentage of the radicals Rc being of formula (C) is ranging from 5% to 100%, preferably from 10% to 100%, based on the total number of the radicals Ra and Rb in said compound of formula (A).

**3.** The polymeric nanoparticle according to claim 2, wherein the percentage of the radicals Rc being a labelling moiety or a targeting moiety is ranging from 1 to 50%, preferably from 20 to 50%, based on the total number of Ra and Rb radicals in said compound of formula (A).

**4.** The polymeric nanoparticle according to claim 2 or 3, wherein when Rc is a group of formula (C), Rc has the following formula (D):

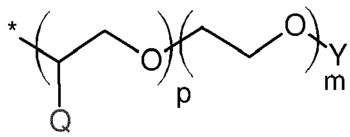

**(D)**

wherein:

p is ranging from 0 to 10, for example p is ranging from 1 to 8, for example p is 3 or 6;
m is ranging from 15 to 300, for example m is ranging from 10 to 50, for example m is raging from 10 to 35;
Q is H or $CH_3$;
wherein * represents the covalent bond to -NH in Rc
wherein Y is as defined in claim 2.

**5.** The polymeric nanoparticle according to any one of claims 2 to 4, wherein said polymeric molecule is a polyethylene glycolated poly(maleic anhydride-alt-1-octadecene).

**6.** The polymeric nanoparticle according to any one of claims 2 to 5, wherein the radicals Rc, independently of each other, are:

- a fluorophore moiety; or
- have the following group of formula (C):

-X-Y (C),

wherein X and Y are as defined in claim 2,
wherein said fluorophore moiety and said group of formula (C) are preferably both present in said polymeric molecule.

**7.** The polymeric nanoparticle according to claim 6, wherein said fluorophore moiety is selected from the group consisting of:

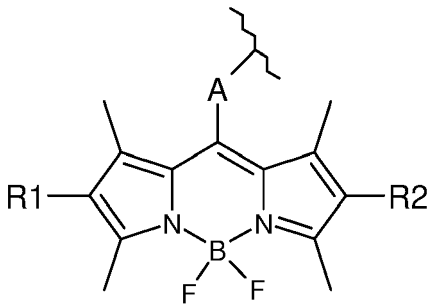

wherein A is a chain comprising carbon atoms and one or more heteroatoms, such as one or more chemical functions -NH-, -O-, -C(=O)-, -C(=O)-NH-, and optionally comprising one or more aromatic groups either in the main chain or as pendant groups,
wherein R1 and R2 are each independently H, an alkyl group, an aromatic group, an alkylaryl group or an aralkyl group, such as for example a methyl, ethyl of phenyl or phenylphenyl group,
wherein

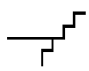

represents a covalent bond to -NH group of formula (B).

**8.** The polymeric nanoparticle according to claim 6 or 7, wherein the fluorophore moiety is a cationic fluorophore moiety, preferentially rhodamine or a cyanine, with a counter-ion.

**9.** The polymeric nanoparticle according to any one of claims 6 to 8, wherein the percentage of the radicals Rc is a fluorophore ranging from 1% to 50%, preferably from 10% to 50%, based on the total number of the radicals Ra and Rb in said compound of formula (A).

**10.** The polymeric nanoparticle according to any one of claims 2 to 9, wherein:

the radicals Rc, independently of each other, are:

- a labelling moiety, preferably a fluorophore moiety or
- has the following formula (C):

-X-Y **(C)**

wherein

- X has one of the following formulae: or

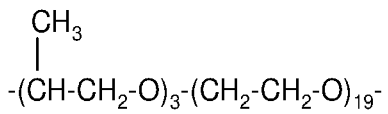

and
- Y is $-NH_2$ or $-CH_3$

wherein the percentage of the radicals Rc being of formula (C) is comprised between 5% and 100%, preferably between 40% and 100%, based on the total number of the radicals Ra and Rb in said compound of formula (A).

**11.** The polymeric nanoparticle according to any one of the preceding claims, wherein the nanoparticles have a mean diameter of less than 35 nm, preferably comprised between 5 nm and 25 nm, more preferably between 10 nm and 20 nm.

**12.** Use of at least one polymeric nanoparticle according to anyone of claims 1 to 11, as a nanoprobe or as a drug delivery system.

**13.** Use of at least one polymeric nanoparticle according to anyone of claims 1 to 11, in bioimaging, cellular imaging or medical imaging.

**14.** An aqueous composition comprising:

- at least one polymeric nanoparticle according to any one of claims 1 to 11;
and
- an aqueous medium, which include but not limited to water, including miliQ water, dionized water, distilled water, buffered water, salted water like PBS.

**15.** The aqueous composition according to claim 14, wherein said composition is an injectable composition.

**16.** A pharmaceutical composition or a composition for use in a method of diagnosis, said composition comprising at least one polymeric nanoparticle according to anyone of claims 1 to 11.

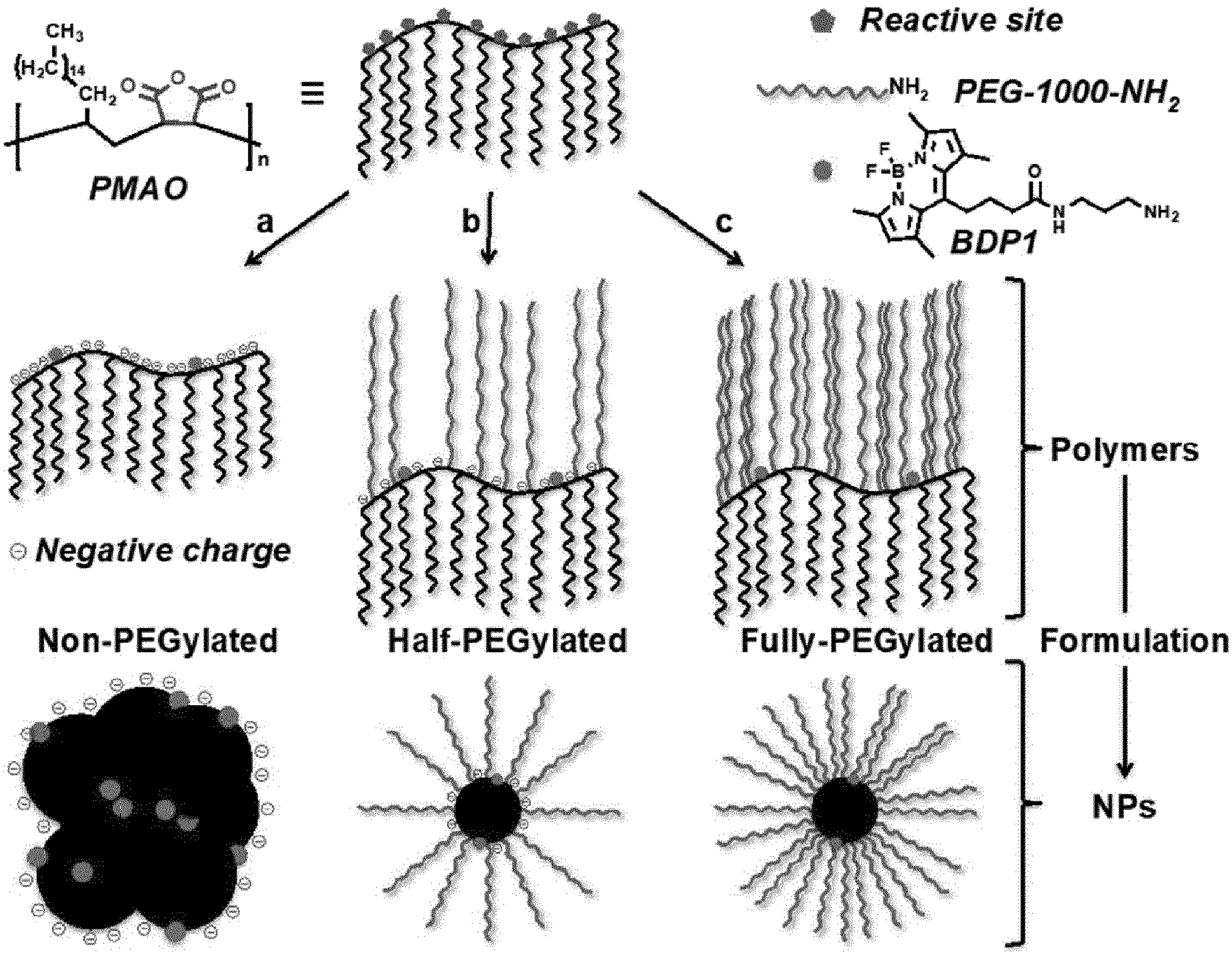
Reactive site
PEG-1000-NH2
PMAO
BDP1
a
b
c
Negative charge
Non-PEGylated
Half-PEGylated
Fully-PEGylated
Polymers
Formulation
NPs

FIG.1

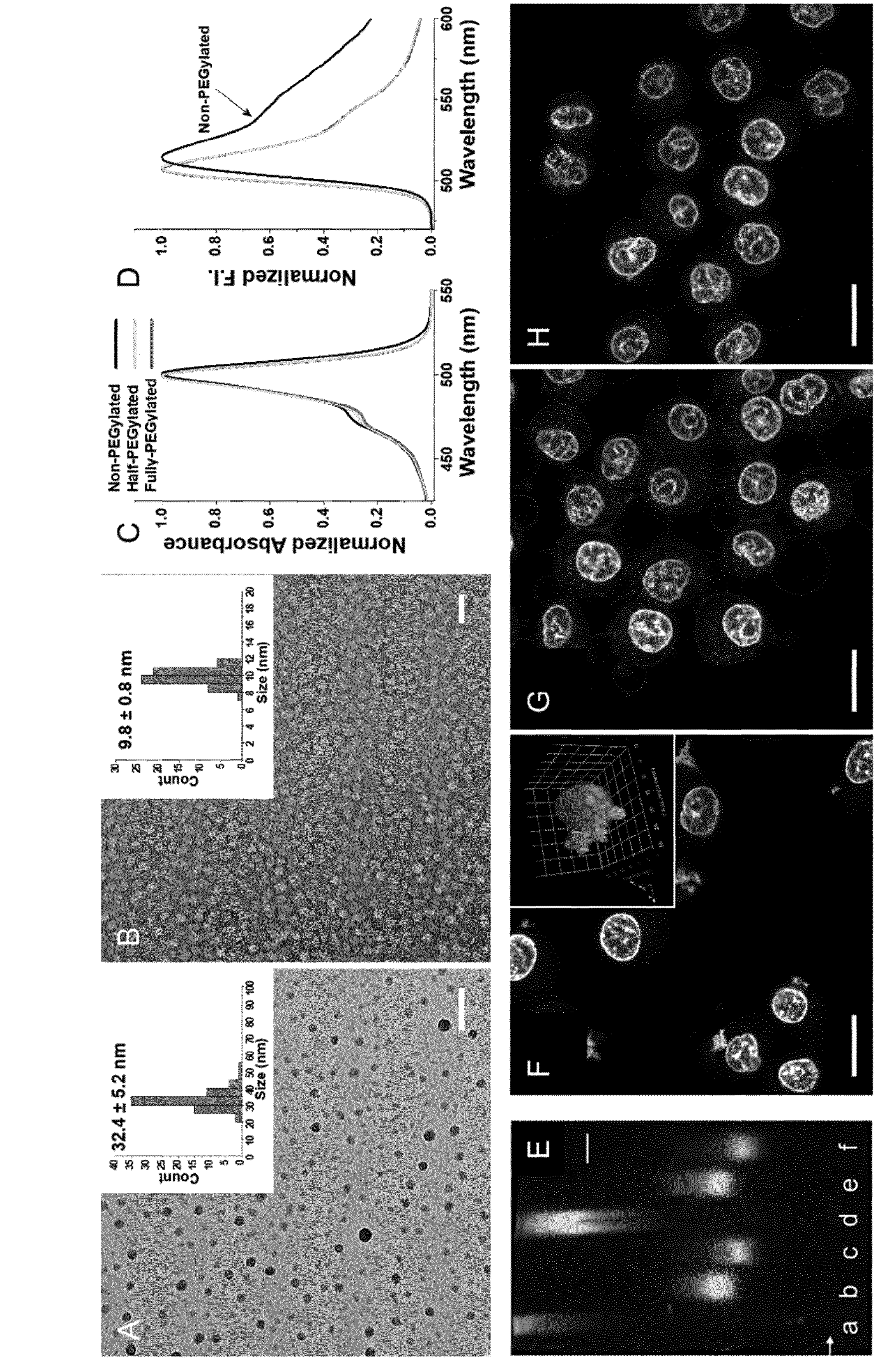
A
32.4 ± 5.2 nm
Count
Size (nm)
B
9.8 ± 0.8 nm
Count
Size (nm)
C
Normalized Absorbance
Wavelength (nm)
Non-PEGylated
Half-PEGylated
Fully-PEGylated
D
Normalized F.I.
Wavelength (nm)
Non-PEGylated
E
a b c d e f
F
G
H

FIG.2

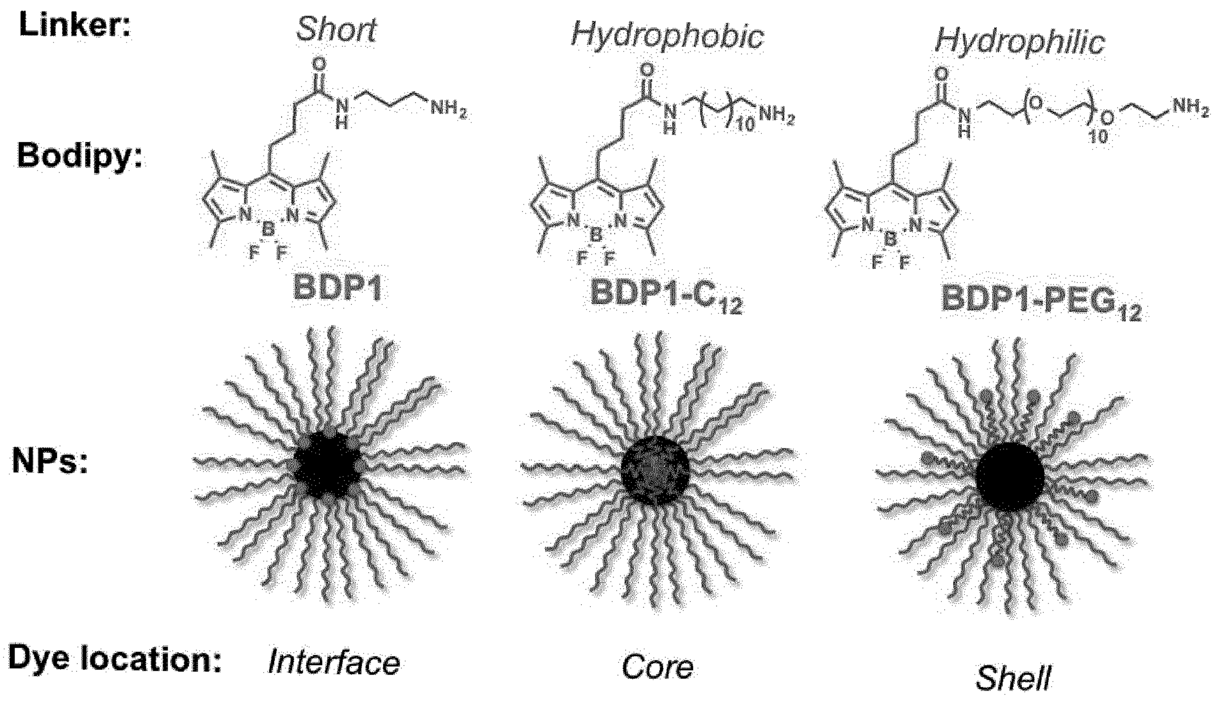
Linker:
Short
Hydrophobic
Hydrophilic
Bodipy:
BDP1
BDP1-$C_{12}$
BDP1-$PEG_{12}$
NPs:
Dye location:
Interface
Core
Shell

FIG.3

FIG.4

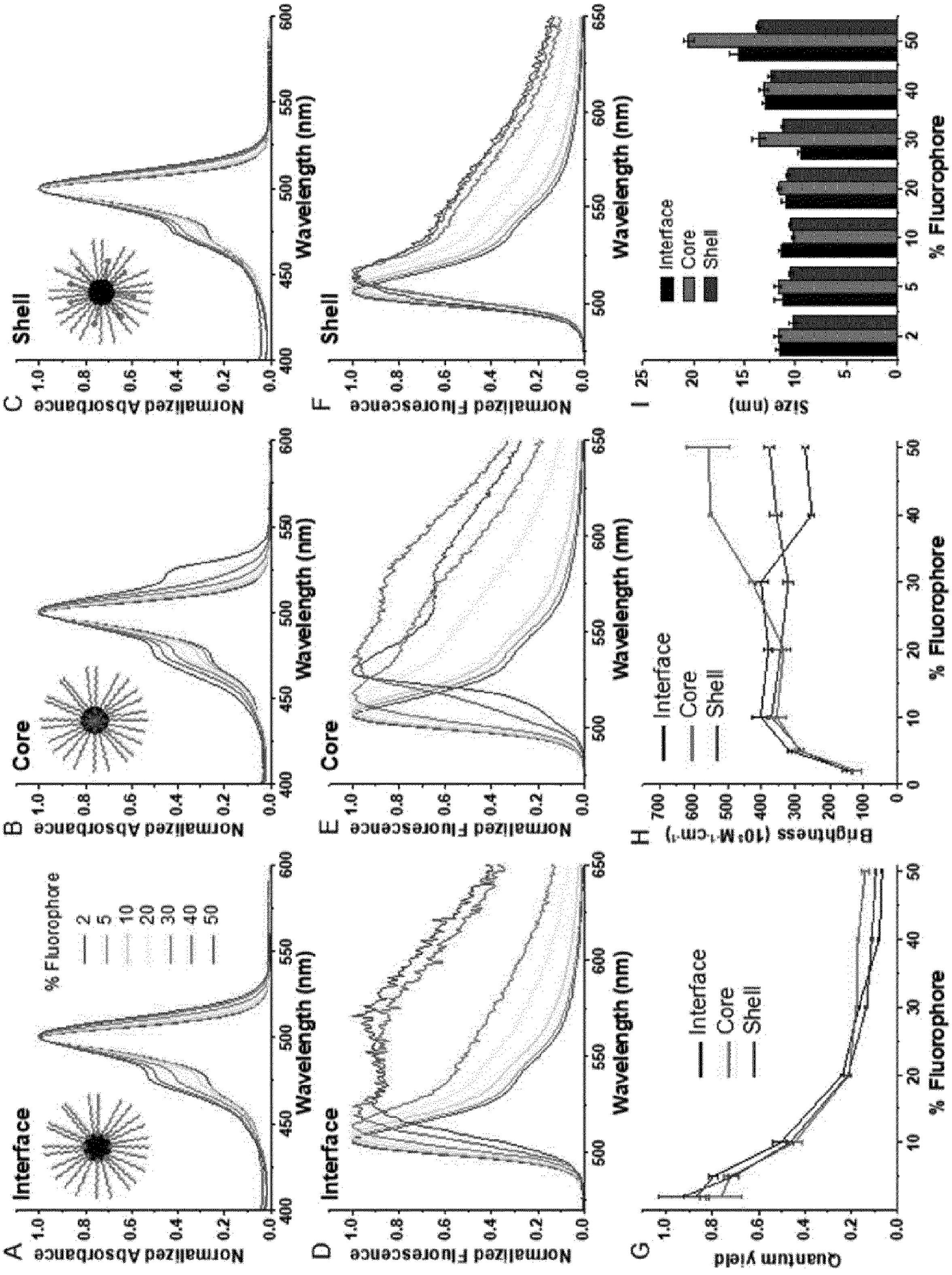
A
Interface
% Fluorophore
2
5
10
20
30
40
50
Normalized Absorbance
Wavelength (nm)
B
Core
C
Shell
D
Interface
E
Core
F
Shell
Normalized Fluorescence
G
Quantum yield
Interface
Core
Shell
% Fluorophore
H
Brightness (10³M⁻¹cm⁻¹)
I
Size (nm)

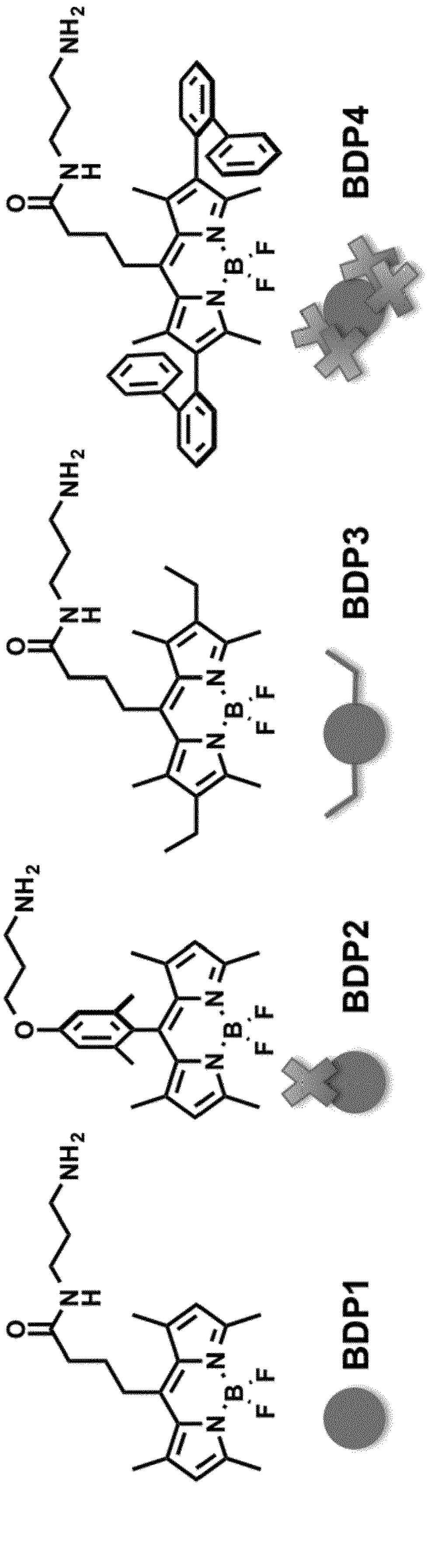

BDP1

BDP2

BDP3

BDP4

FIG.5

FIG.6

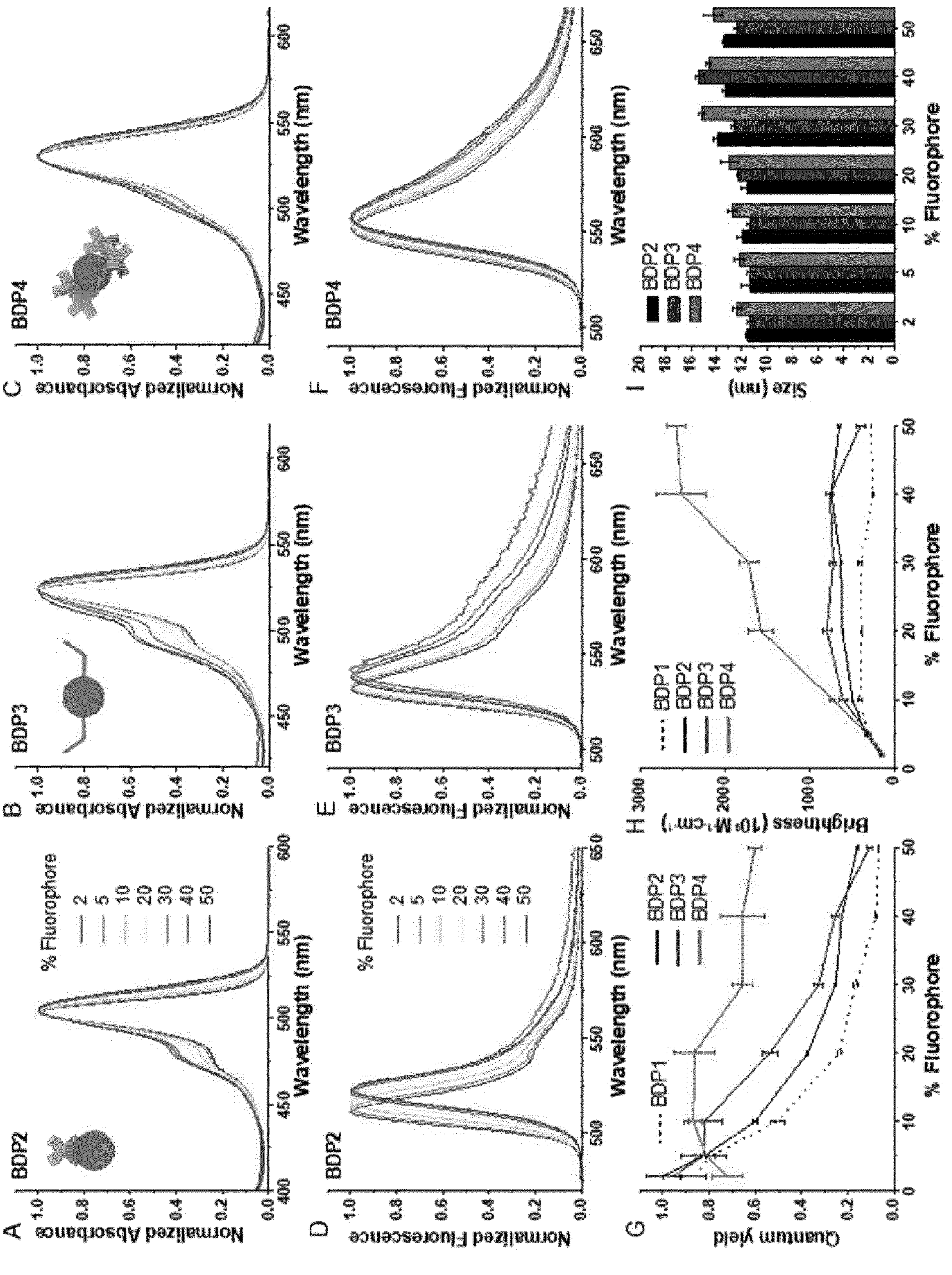
A
BDP2
% Fluorophore
2
5
10
20
30
40
50
Normalized Absorbance
Wavelength (nm)
B
BDP3
C
BDP4
D
E
F
Normalized Fluorescence
G
Quantum yield
BDP1
BDP2
BDP3
BDP4
% Fluorophore
H
Brightness (10³ M⁻¹cm⁻¹)
I
Size (nm)

FIG.7

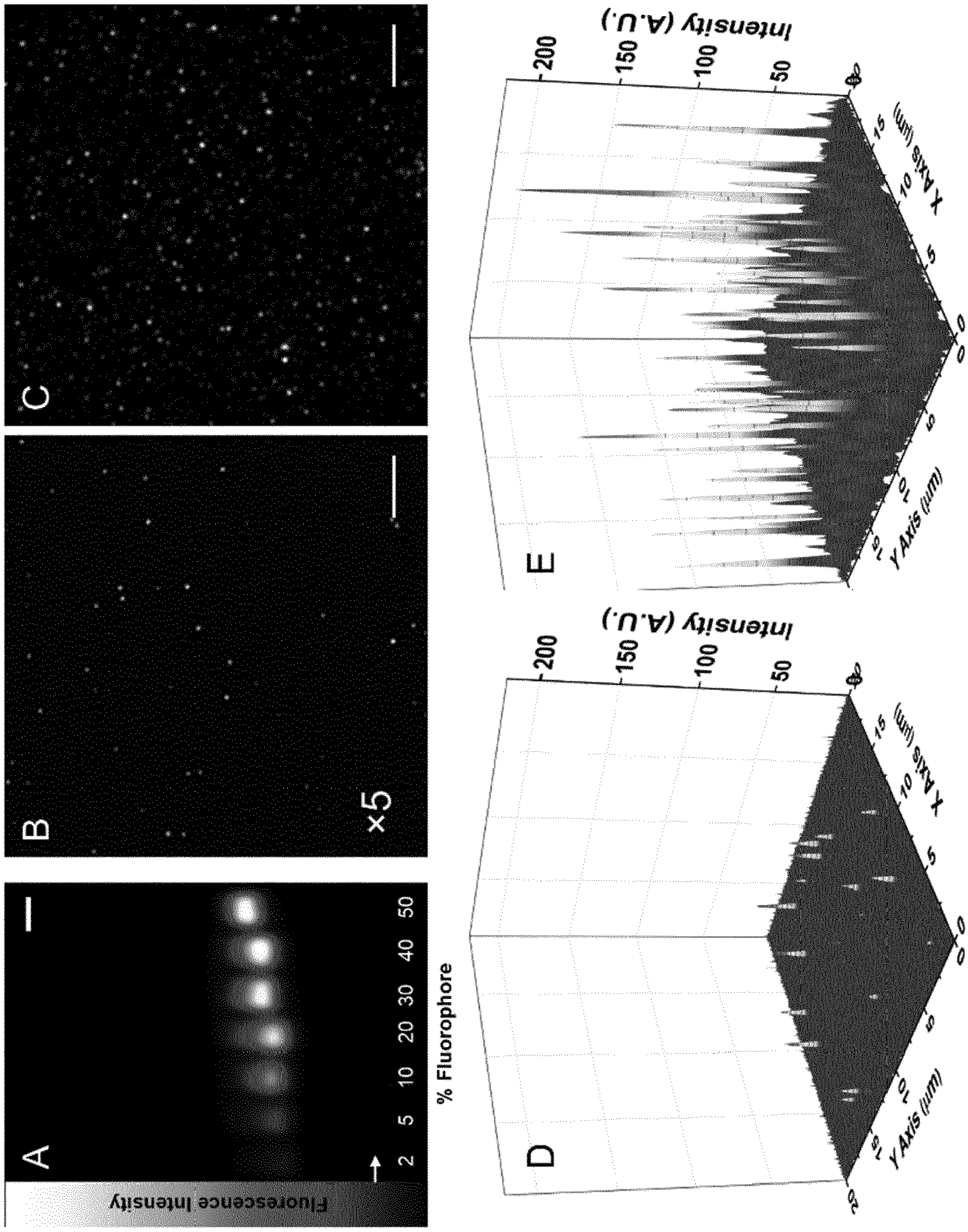
A
B
C
D
E
Fluorescence Intensity
% Fluorophore
2
5
10
20
30
40
50
×5
Intensity (A.U.)
200
150
100
50
0
X Axis (um)
Y Axis (um)
0
5
10
15
20

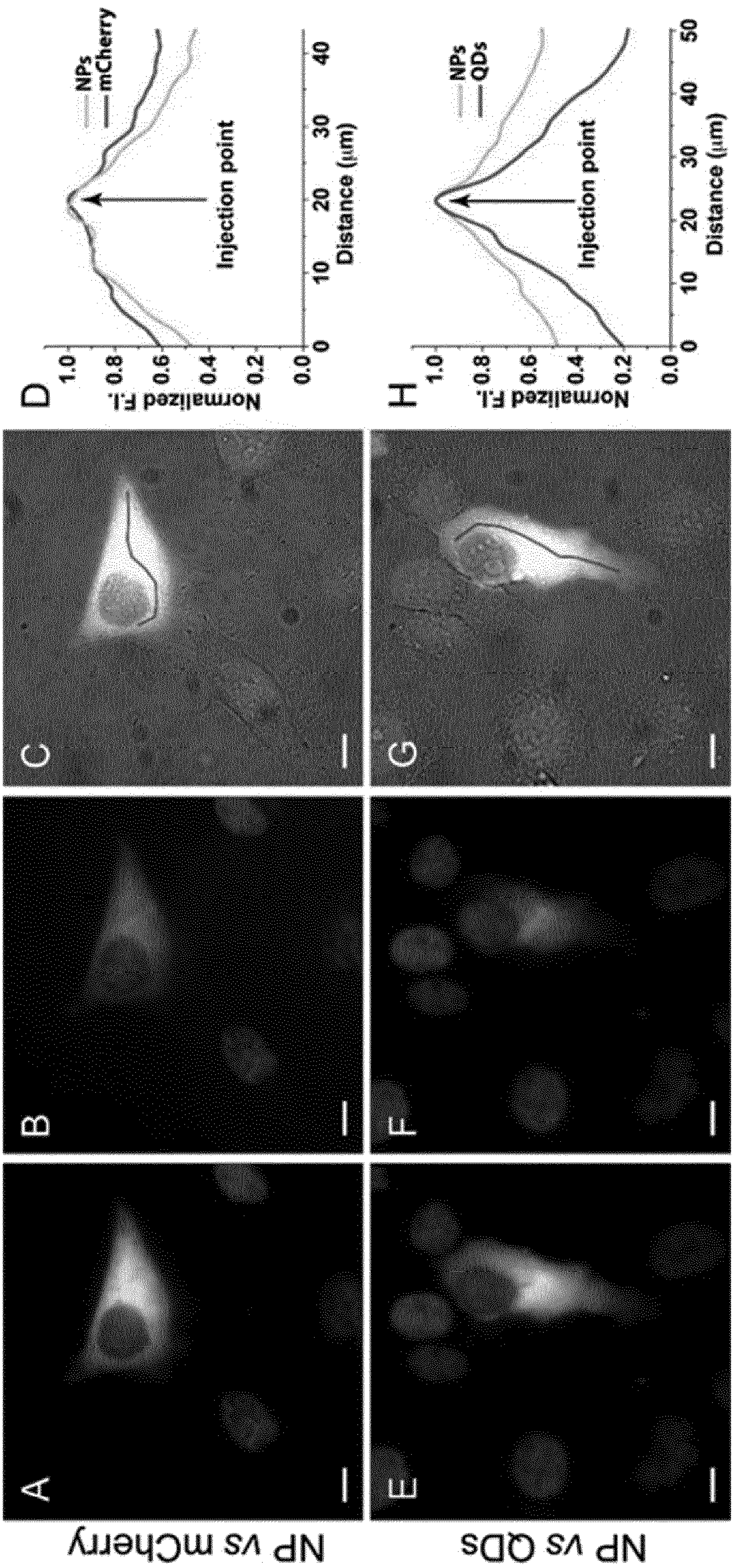
NP vs mCherry
NP vs QDs
A
B
C
D
E
F
G
H
NPs
mCherry
QDs
Injection point
Normalized F.I.
Distance (μm)

FIG.8

FIG.9

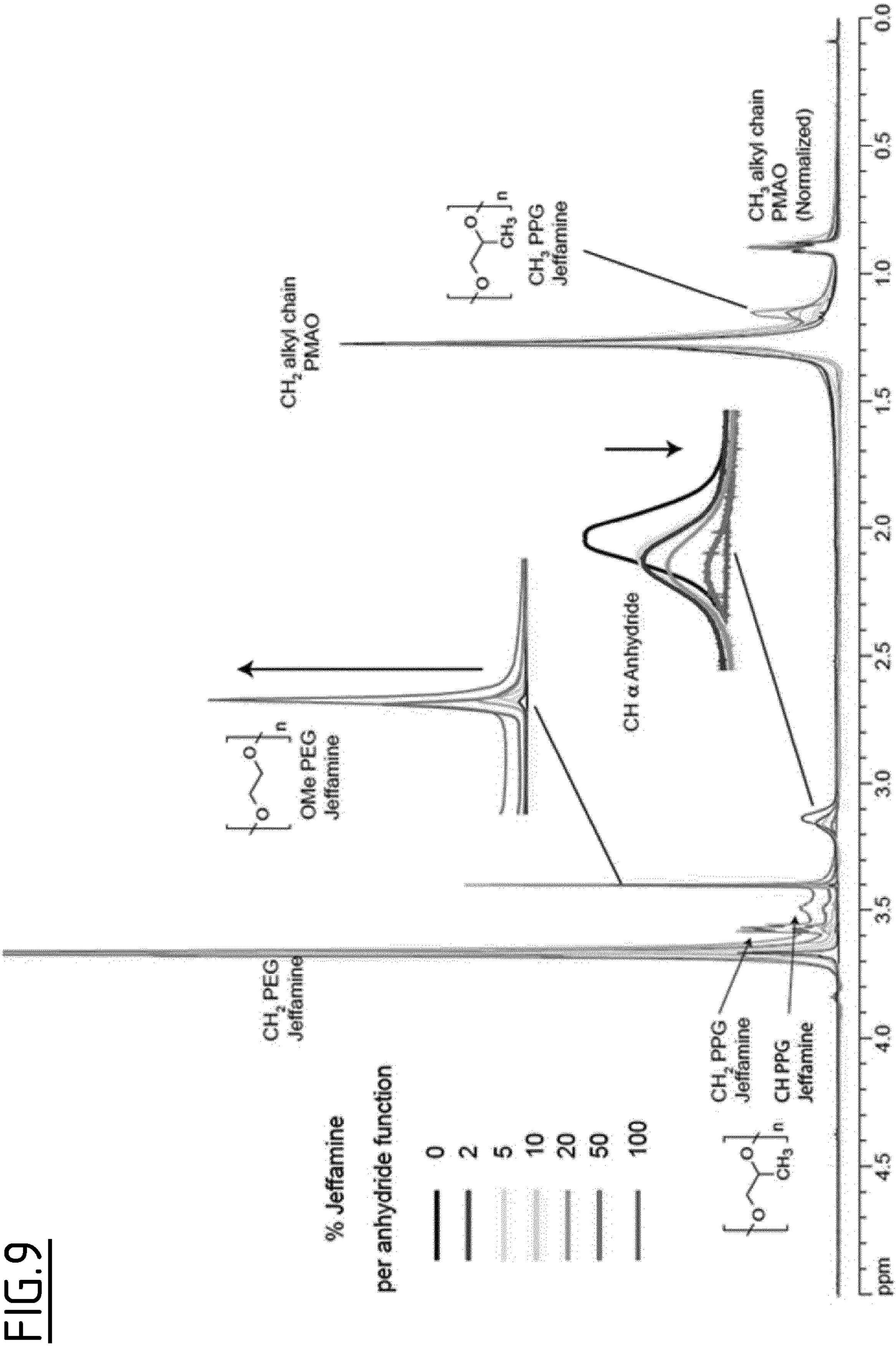

% Jeffamine
per anhydride function
0
2
5
10
20
50
100
CH2 PEG Jeffamine
CH2 PPG Jeffamine
CH PPG Jeffamine
OMe PEG Jeffamine
CH α Anhydride
CH2 alkyl chain PMAO
CH3 PPG Jeffamine
CH3 alkyl chain PMAO (Normalized)
ppm
4.5
4.0
3.5
3.0
2.5
2.0
1.5
1.0
0.5
0.0

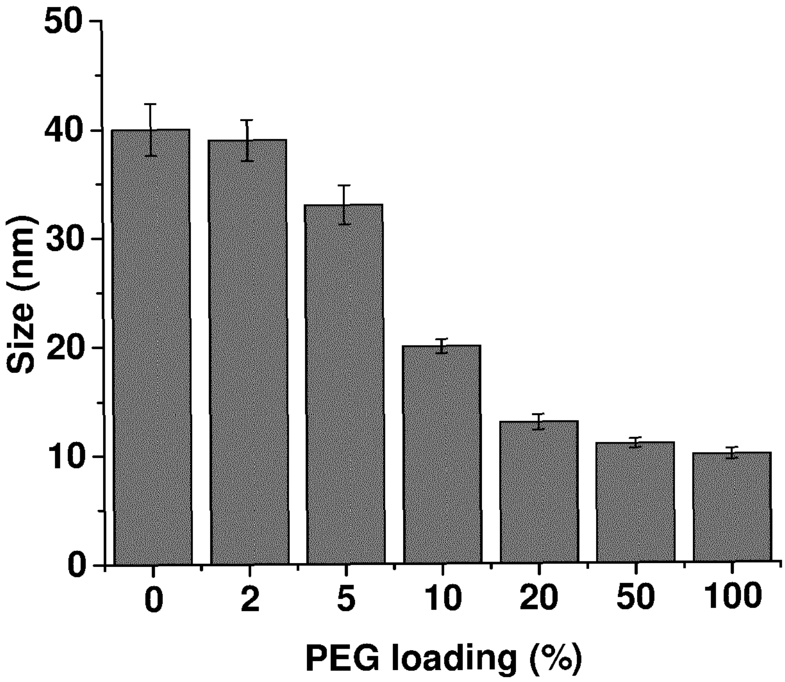


FIG.10

FIG.11

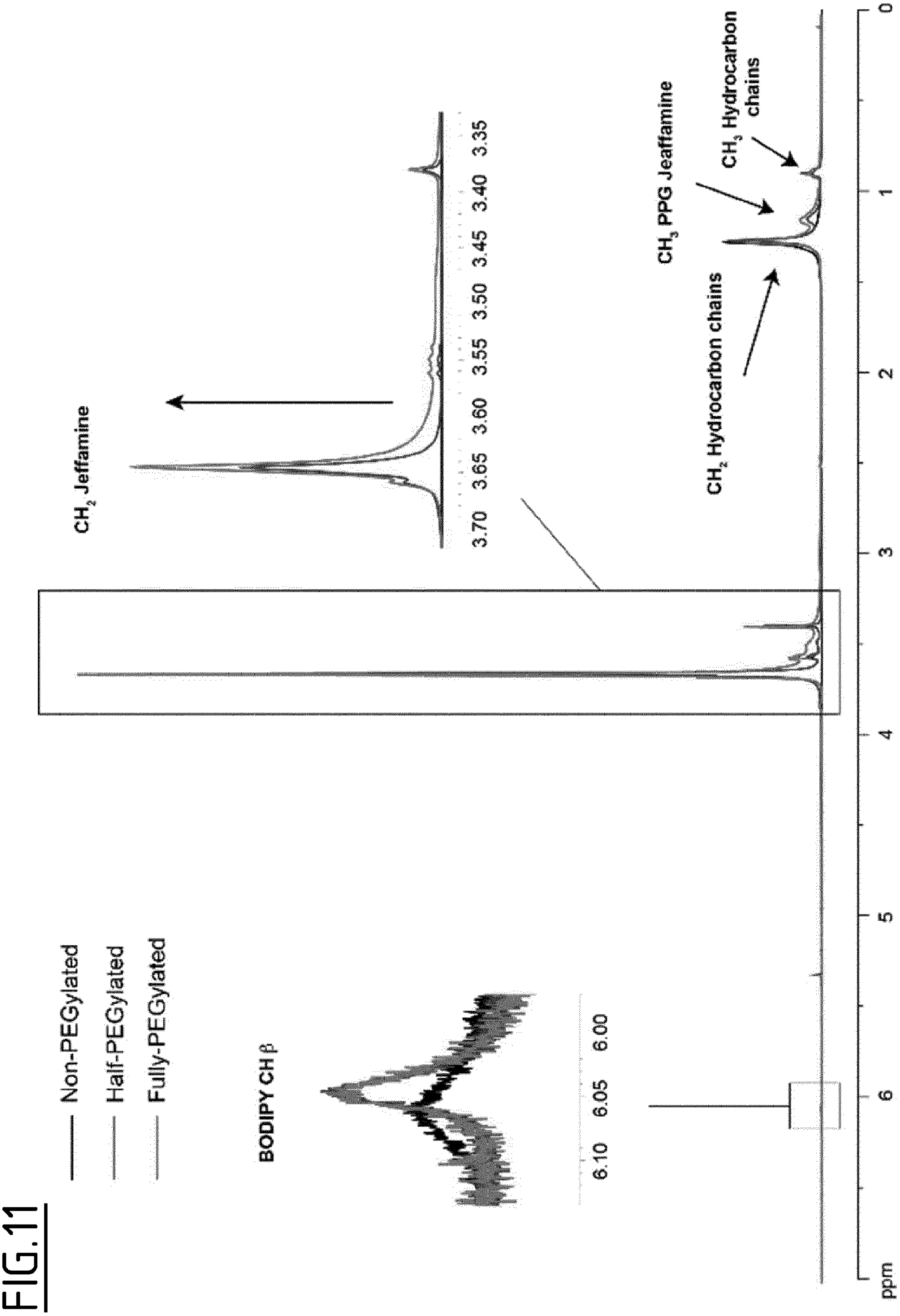
Non-PEGylated
Half-PEGylated
Fully-PEGylated
BODIPY CH β
6.10
6.05
6.00
CH$_2$ Jeffamine
3.70
3.65
3.60
3.55
3.50
3.45
3.40
3.35
CH$_2$ Hydrocarbon chains
CH$_3$ PPG Jeaffamine
CH$_3$ Hydrocarbon chains
ppm
6
5
4
3
2
1
0

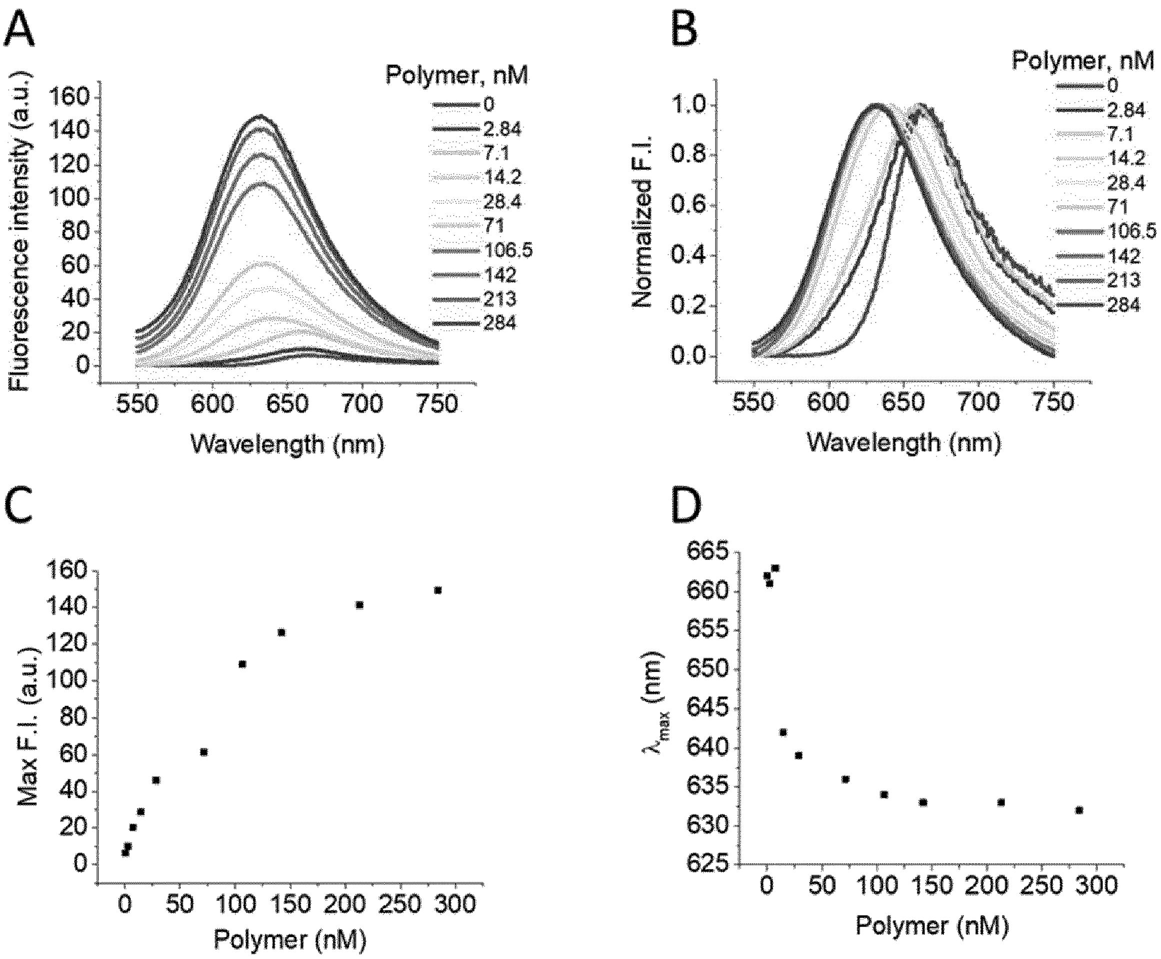
A
Polymer, nM
0
2.84
7.1
14.2
28.4
71
106.5
142
213
284
Fluorescence intensity (a.u.)
Wavelength (nm)
B
Polymer, nM
Normalized F.I.
Wavelength (nm)
C
Max F.I. (a.u.)
Polymer (nM)
D
$\lambda_{max}$ (nm)
Polymer (nM)

FIG.12

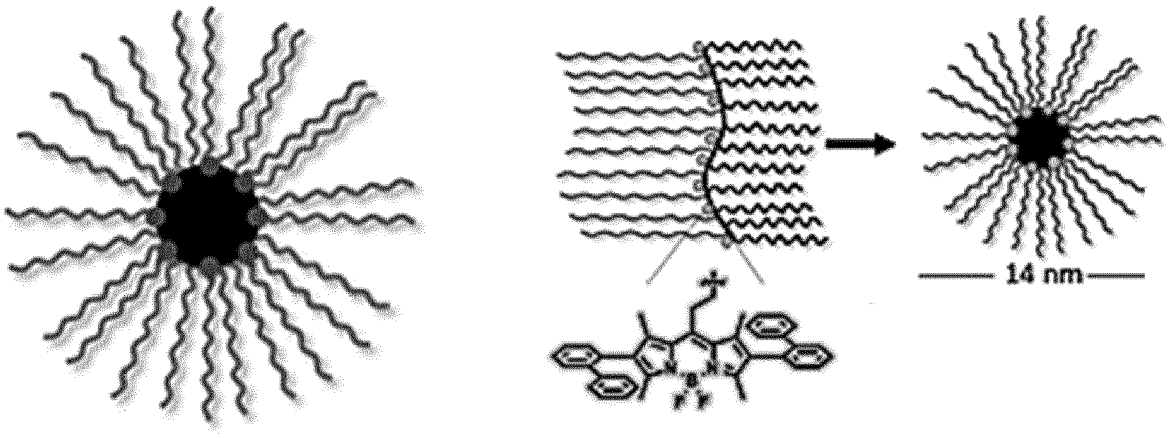


(N)

FIG.13

Europäisches Patentamt
European Patent Office
Office européen des brevets

# EUROPEAN SEARCH REPORT

Application Number
EP 20 30 6027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOU SOPHIE ET AL: "Lipid-core/polymer-shell hybrid nanoparticles: synthesis and characterization by fluorescence labeling and electrophoresis", SOFT MATTER, vol. 16, no. 17, 1 April 2020 (2020-04-01), pages 4173-4181, XP055775873, ISSN: 1744-683X, DOI: 10.1039/D0SM00077A Retrieved from the Internet: URL:https://pubs.rsc.org/en/content/articlepdf/2020/sm/d0sm00077a> * figure 3, formulation 4 * * figure 4 * ----- | 1-16 | INV. A61K49/00 G01N21/64 |
| X,P | COLLOT MAYEUL ET AL: "Stealth and Bright Monomolecular Fluorescent Organic Nanoparticles Based on Folded Amphiphilic Polymer", ACS NANO, vol. 14, no. 10, 6 October 2020 (2020-10-06), pages 13924-13937, XP055775924, ISSN: 1936-0851, DOI: 10.1021/acsnano.0c06348 Retrieved from the Internet: URL:https://pubs.acs.org/doi/pdf/10.1021/acsnano.0c06348> * the whole document * ----- | 1-16 | TECHNICAL FIELDS SEARCHED (IPC): A61K G01N |
| A,D | ANDREAS REISCH ET AL: "Fluorescent Polymer Nanoparticles Based on Dyes: Seeking Brighter Tools for Bioimaging", SMALL, vol. 12, no. 15, 1 April 2016 (2016-04-01), pages 1968-1992, XP055700865, ISSN: 1613-6810, DOI: 10.1002/smll.201503396 * the whole document * ----- | 1-16 | |

1

The present search report has been drawn up for all claims

| Place of search | Date of completion of the search | Examiner |
|---|---|---|
| The Hague | 18 February 2021 | Bliem, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CABRAL, H. ; MATSUMOTO, Y. ; MIZUNO, K. ; CHEN, Q. ; MURAKAMI, M. ; KIMURA, M. ; TERADA, Y. ; KANO, M. R. ; MIYAZONO, K. ; UESAKA, M.** Accumulation of sub-100 nm polymeric micelles in poorly permeable tumours depends on size. *Nature Nanotechnology,* 2011, vol. 6, 815
- **ZHANG, C. ; LI, C. ; LIU, Y. ; ZHANG, J. ; BAO, C. ; LIANG, S. ; WANG, Q. ; YANG, Y. ; FU, H. ; WANG, K.** Gold Nanoclusters-Based Nanoprobes for Simultaneous Fluorescence Imaging and Targeted Photodynamic Therapy with Superior Penetration and Retention Behavior in Tumors. *Adv. Funct. Mater.,* 2015, vol. 25, 1314
- **DI CORATO, R. ; QUARTA, A. ; PIACENZA, P. ; RAGUSA, A. ; FIGUEROLA, A. ; BUONSANTI, R. ; CINGOLANI, R. ; MANNA, L. ; PELLEGRINO, T.** Water solubilization of hydrophobic nanocrystals by means of poly(maleic anhydride-alt-1-octadecene). *J. Mater. Chem.,* 2008, vol. 18, 1991
- **REISCH, A. ; KLYMCHENKO, A. S.** Fluorescent Polymer Nanoparticles Based on Dyes: Seeking Brighter Tools for Bioimaging. *Small,* 2016, vol. 12, 1968
- **REISCH, A. ; DIDIER, P. ; RICHERT, L. ; ONCUL, S. ; ARNTZ, Y. ; MELY, Y. ; KLYMCHENKO, A. S.** Collective fluorescence switching of counterion-assembled dyes in polymer nanoparticles. *Nat. Commun.,* 2014, vol. 5, 4089